# EUROPEAN PATENT APPLICATION

(11) **EP 4 721 740 A1**
(43) Date of publication of application: **08.04.2026**
(21) Application number: 24814566.6
(22) Date of filing: 31.05.2024
(51) Int. Cl.: A61K 31/4745, A61K 31/575, A61P 25/28, A61P 25/14, A61P 25/16, A61P 21/00

(54) **COMBINATION PRODUCT, SALT, AND USE THEREOF IN TREATING NEURODEGENERATIVE DISEASES OR DISORDERS**

(30) Priority: 31.05.2023 CN 202310639549; 15.03.2024 CN 202410302178
(71) Applicant: Shanghai Henlius Biotech, Inc., Shanghai 201210 (CN); Shanghai Henlius Biologics Co., Ltd., Shanghai 201616 (CN)
(72) Inventor: SHAN, Yongqiang, Shanghai 201210 (CN); LV, Zhiliang, Shanghai 201210 (CN); FENG, Teng, Shanghai 201210 (CN)
(74) Representative: Rentsch Partner AG
(86) International application number: PCT/CN2024/096520
(87) International publication number: WO 2024/245371

(57) **Abstract**

A novel combination product, a pharmaceutical composition, a salt form, a preparation method therefor and a use thereof in treating, relieving and/or preventing neurodegenerative diseases or disorders.

## Description

### TECHNICAL FIELD

The present disclosure pertains to the field of pharmaceuticals, and relates to a novel combination product, a pharmaceutical composition, a salt form, a preparation method therefor, and use thereof in the treatment, alleviation, and/or prevention of a neurodegenerative disease or disorder.

### BACKGROUND

Alzheimer's disease (AD) is a chronic neurodegenerative disease characterized by neuronal cell death, loss of cognitive ability, severe behavioral abnormalities, ultimately leading to the patient's death. Currently, there are 2.5 to 4 million AD patients in the United States and 17 to 25 million AD patients worldwide. This disease has become the fourth leading cause of death in Western countries, following heart disease, cancer, and stroke. ARICEPT^{®} is an acetylcholinesterase inhibitor that has been approved by the FDA for slowing the rate of exacerbation in patients with Alzheimer's disease. However, this drug is only effective during a limited period and for certain patients. To date, there is no definitive drug that can treat or cure this devastating disease.

Amyotrophic lateral sclerosis (ALS) is a severe neurodegenerative disease that affects the upper and lower motor neurons in the brainstem and spinal cord. ALS patients experience extensive muscle atrophy 3-5 years after onset, leading to paralysis and death; its pathological characteristics are the aggregation and accumulation of ubiquitinated protein inclusions in motor neurons. ALS can be divided into familial ALS (fALS), accounting for about 10%, and sporadic ALS (sALS) without a family history of the disease, accounting for about 90%. The main contributing factors of ALS include genetics, environment, lifestyle, etc., or the interaction therebetween (PARALS Registry et al., "Genome-Wide Association Analyses Identify New Risk Variants and the Genetic Architecture of Amyotrophic Lateral Sclerosis."). In terms of pathogenesis, ALS involves multiple pathophysiological pathways, including glutamate excitotoxicity (Van Den Bosch et al., "The Role of Excitotoxicity in the Pathogenesis of Amyotrophic Lateral Sclerosis."), neuroinflammation (Liu and Wang, "Role of Neuroinflammation in Amyotrophic Lateral Sclerosis."), iron accumulation (Ndayisaba, Kaindlstorfer, and Wenning, "Iron in Neurodegeneration - Cause or Consequence?"), dysregulated microRNA metabolism and abnormal RNA-binding proteins (Donnelly, Grima, and Sattler, "Aberrant RNA Homeostasis in Amyotrophic Lateral Sclerosis."), etc. Its pathogenesis has not yet been fully elucidated.

The incidence and prevalence of ALS increase with age. To date, there is no treatment or medication that can halt or reverse this disease; approved drugs are intended to alleviate symptoms or slow disease progression. Since its approval by the U.S. Food and Drug Administration (FDA) in 1995, riluzole has been the only widely recognized drug for the treatment of ALS, extending patients' survival by 3 months (Miller, Mitchell, and Moore, "Riluzole for Amyotrophic Lateral Sclerosis (ALS)/Motor Neuron Disease (MND)."). In 2017, the U.S. Food and Drug Administration (FDA) approved Evadarone, which improves the revised ALS functional rating scale (ALSFRS-R) score and shows a trend of slowing disease progression (Al-Chalabi et al., "July 2017 ENCALS Statement on Edaravone."). In September 2022, AMX0035 was approved by the FDA for the treatment of ALS. In the ITT trial, the median overall survival (median OS) was extended by 4.8 months, and then adjustment was performed by two post-hoc analysis methods; the RPSFTM-adjusted extension was 9.7 months, and a survival prediction algorithm created from natural history data indicated an extension of 9.9 months

in survival benefit. AMX0035 is an oral fixed-dose combination of sodium phenylbutyrate (PB) and tauroursodeoxycholic acid (TUDCA) developed by Amyyx Pharmaceuticals. Sodium phenylbutyrate is a histone deacetylase inhibitor that can inhibit endoplasmic reticulum stress response; tauroursodeoxycholic acid can inhibit mitochondria-associated apoptosis (Mead et al., "Amyotrophic Lateral Sclerosis."). AMX0035 is intended to target the endoplasmic reticulum (ER) and mitochondria of motor neurons in ALS. The endoplasmic reticulum and mitochondria are connected by the mitochondrial membrane, and both play a key role in maintaining neuronal survival.

Parkinson's disease (PD) is a chronic neurodegenerative disease that primarily affects the elderly. Its most common clinical features are motor tremors (a rhythmic shaking), muscle rigidity, and slowed movement (known as bradykinesia), but it may also present with non-motor symptoms, including sleep disturbances, constipation, anxiety, depression, fatigue, etc. Its main pathological features are the loss of dopaminergic neurons in the substantia nigra pars compacta (SNpc) of the midbrain and dopaminergic nerve fibers in the striatal region, as well as the formation of intraneuronal protein inclusion bodies known as Lewy bodies (LBs), which are primarily composed of α-synuclein (John van Geest Centre for Brain Repair, Department of Clinical Neurosciences, University of Cambridge, UK et al., "Parkinson's Disease."). Parkinson's disease is multifactorial, caused by genetics, environment, or the interaction between the two (Bloem, Okun, and Klein, "Parkinson's Disease."). Parkinson's disease is divided into two forms: sporadic (late-onset) and familial (early-onset) (Ryan et al., "Mitochondrial Dysfunction and Mitophagy in Parkinson's."). Epidemiological studies have shown that familial Parkinson's disease accounts for only a small proportion of Parkinson's disease cases, while the vast majority of Parkinson's disease patients are sporadic Parkinson's disease patients (Zeng, Geng, and Jia, "Neurotoxin-Induced Animal Models of Parkinson Disease."). From 1990 to 2016, the total number of PD patients exceeded 6 million (Dorsey et al., "Global, Regional, and National Burden of Parkinson's Disease, 1990-2016."), which is projected to double to over 12 million by 2040 (Rossi et al., "Projection of the Prevalence of Parkinson's Disease in the Coming Decades."). The etiology of Parkinson's disease remains unclear but may be associated with various risk factors such as excitotoxicity, oxidative stress, and neuroinflammation.

Currently, treatments for PD include levodopa, dopamine agonists, monoamine oxidase (MAO) inhibitors, and amantadine, which aim to ameliorate symptoms and slow disease progression. However, these treatment methods cannot completely cure the disease and also have some side effects.

Existing therapies for neurodegenerative diseases such as Alzheimer's disease (AD), amyotrophic lateral sclerosis (ALS), and Parkinson's disease (PD) face numerous challenges. ARICEPT^{®} is only effective during a limited period and for certain patients with AD. The treatments of ALS are usually costly and partially ineffective; current therapeutic drugs or measures can only achieve limited amelioration, posing a significant unmet clinical need. Parkinson's disease has a great social impact, and is currently the fastest-growing nervous system disease and a leading cause of disability worldwide. Current therapeutic drugs or measures aim to ameliorate symptoms and slow disease progression, but also come with many side effects, so there is still a substantial unmet clinical need.

Therefore, given the complex pathogenesis of neurodegenerative diseases or disorders, there is an urgent need in the art for a novel therapy for neurodegenerative diseases or disorders that offers improved efficacy and higher safety compared to existing therapies.

### SUMMARY

The inventors of the present application have addressed the above needs through creative work.

Specifically, the present disclosure relates to the following technical solutions:
In one embodiment, the present application relates to a combination product, which comprises:
(a) a compound of formula (I) or a pharmaceutically acceptable salt, a solvate, a hydrate, or a stereoisomer thereof: 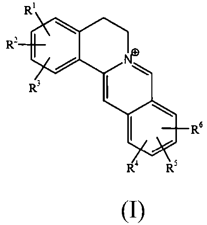
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N; and
(b) a bile acid or a derivative or an analog thereof, or a pharmaceutically acceptable salt, a solvate, a hydrate, or a stereoisomer thereof;
   wherein the molar ratio of the component (a) to the component (b) is 1:1000-1000:1.

In another embodiment, the present disclosure relates to a pharmaceutical composition, which comprises:
(a) a compound of formula (I) or a pharmaceutically acceptable salt, a solvate, a hydrate, or a stereoisomer thereof: 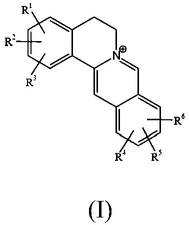 wherein R¹, R², R³, R⁴, R⁵, and R⁶ are as defined herein;
(b) a bile acid or a derivative or an analog thereof, or a pharmaceutically acceptable salt, a solvate, a hydrate, or a stereoisomer thereof; and
(c) a pharmaceutically acceptable carrier, excipient, and/or diluent;
   wherein the molar ratio of the component (a) to the component (b) is 1:1000-1000:1.

In yet another embodiment, the present disclosure relates to an acid-base addition salt of formula (II):

(A⁺)ₘ(B⁻)ₙ(C⁻)ₚ (II)

wherein
(a) A⁺ is a cationic moiety, which is a compound of formula (I): 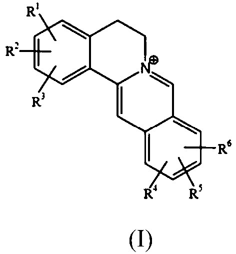 wherein R¹, R², R³, R⁴, R⁵, and R⁶ are as defined herein;
(b) B⁻ is an anionic moiety, which is a bile acid or a derivative or an analog thereof; and
(c) C⁻ is an acid anion;
   wherein m, n, and p are each independently an integer selected from 1-6, such that the configuration of the salt reaches charge balance, and when m = n, p is 0.

In yet another embodiment, the present disclosure relates to the combination product, the pharmaceutical composition, the acid-base addition salt, or the compound of formula (I) or the pharmaceutically acceptable salt, the solvate, the hydrate, or the stereoisomer thereof according to any one of the preceding embodiments for use in improving the viability of cells, particularly neuronal cells, especially by reducing active oxidative metabolite-mediated oxidative damage in the cells, modulating redox homeostasis in the cells, or reducing mitochondrial dysfunction in the cells.

In yet another embodiment, the present disclosure relates to the combination product, the pharmaceutical composition, the acid-base addition salt, or the compound of formula (I) or the pharmaceutically acceptable salt, the solvate, the hydrate, or the stereoisomer thereof according to any one of the preceding embodiments for use in treating, alleviating, and/or preventing a neurodegenerative disease or disorder.

In yet another embodiment, the present disclosure relates to a method for improving the viability of cells, particularly neuronal cells, especially by reducing active oxidative metabolite-mediated oxidative damage in the cells, modulating redox homeostasis in the cells, or reducing mitochondrial dysfunction in the cells, which comprises contacting the cells with the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In still another embodiment, the present disclosure relates to a method for treating, alleviating, and/or preventing a neurodegenerative disease or disorder, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In yet still another embodiment, the present disclosure relates to a kit, which comprises:
the combination product, the acid-base addition salt, the pharmaceutical composition, or the compound of formula (I) or the pharmaceutically acceptable salt, the solvate, the hydrate, or the stereoisomer thereof according to any one of the preceding embodiments, and instructions for use,
wherein the kit is used for treating and/or preventing a neurodegenerative disease or disorder.

The details of the present application are set forth in the accompanying description below. Although methods and materials similar or equivalent to those described herein can be used in the practice or testing of the present application, the illustrative methods and materials are now described. Other features, objectives, and advantages of the present application will be apparent from the specification and the claims. In the specification and the appended claims, the singular forms also include their plural forms, unless the context clearly dictates otherwise. Unless otherwise defined, all technical and scientific terms used herein have the same meanings as commonly understood by those of ordinary skill in the art to which the present application belongs. All patents and publications cited in this specification are incorporated herein by reference in their entirety.

The content of all references (including literature references, issued patents, published patent applications, and co-pending patent applications) cited throughout the present application is hereby expressly incorporated by reference in its entirety. Unless otherwise defined, all technical and scientific terms used herein have the meanings commonly known to those of ordinary skill in the art.

### BRIEF DESCRIPTION OF THE DRAWINGS

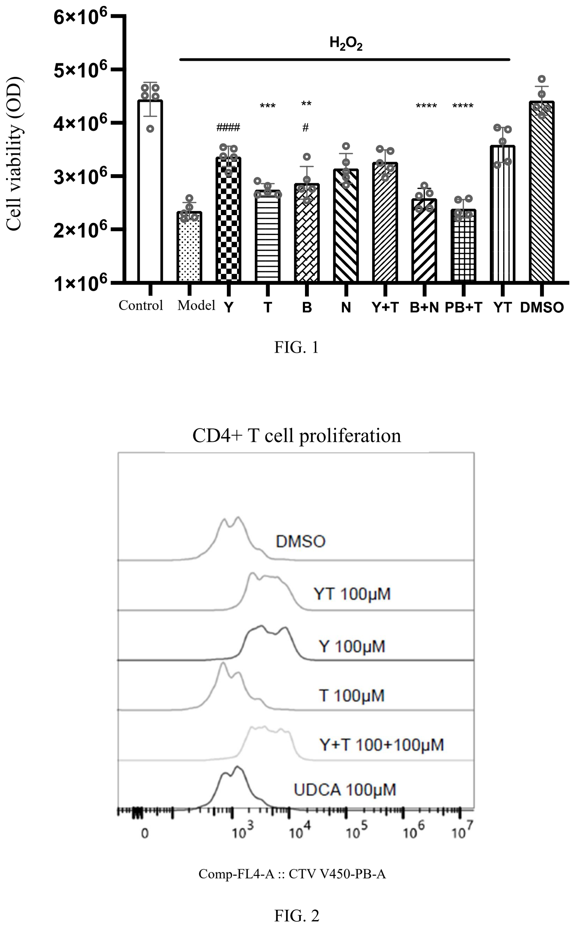

FIG. 1 depicts the effects of compound Y, compound T, compound B, compound N, a compound combination Y + T, a compound combination B + N, the compound combination PB + T, and a novel salt YT on the viability of hydrogen peroxide-induced SH-sy5y cells. In the figure, the control corresponds to a cell culture group without any drug treatment and without H₂O₂ stimulation treatment; the model corresponds to a cell culture group without any drug treatment but with H₂O₂ stimulation treatment; DMSO corresponds to a cell culture group without any drug treatment and without H₂O₂ stimulation treatment but with the addition of DMSO only. In the treatment groups, the concentration of compound Y was 100 µM; the concentration of compound T was 100 µM; the concentration of compound B was 100 µM; the concentration of compound N was 100 µM; the concentration of the compound combination Y + T was 100 µM (compound Y) + 100 µM (compound T); the concentration of the compound combination B + N was 100 µM (compound B) + 100 µM (compound N); the concentration of the compound combination PB + T was 500 µM (compound T) + 200 µM (compound PB); the concentration of the YT salt was 100 µM. All treatment groups were subjected to stimulation induction using H₂O₂. Data are presented as mean ± S.E.M. One-way ANOVA was used. **** indicates P < 0.0001, *** indicates P < 0.001, ** indicates P < 0.01, * indicates P < 0.05, vs. YT; #### indicates P < 0.0001, # indicates P < 0.05, vs. model.
FIG. 2 depicts the effects of compound Y, compound T, the compound combination Y + T, the YT salt, and compound UDCA (U) on the proliferation of CD4-positive T cells. In the experiment, the concentration of the YT salt was 100 µM, which corresponded to proportionally equivalent concentrations of 100 µM for compound Y, 100 µM for compound T, and 100 µM (compound Y) +100 µM (compound T) for the compound combination Y + T; the concentration of compound U was 100 µM.

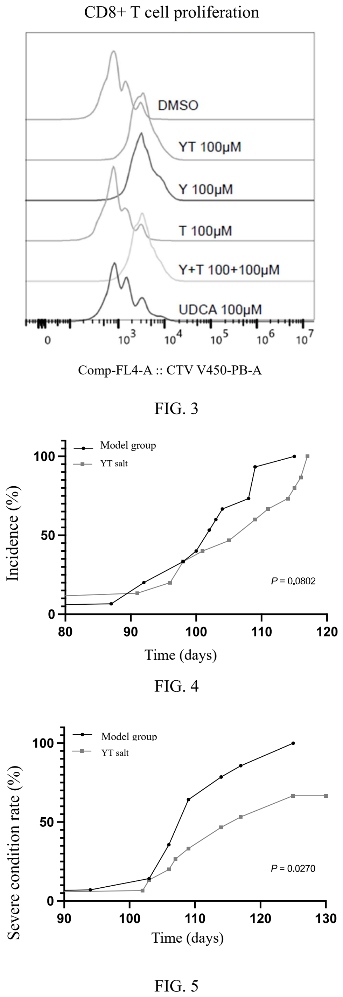

FIG. 3 depicts the effects of compound Y, compound T, the compound combination Y + T, the YT salt, and compound UDCA (U) on the proliferation of CD8-positive T cells. In the experiment, the concentration of the YT salt was 100 µM, which corresponded to proportionally equivalent concentrations of 100 µM for compound Y, 100 µM for compound T, and 100 µM (compound Y) +100 µM (compound T) for the compound combination Y + T; the concentration of compound U was 100 µM.
FIG. 4 depicts the effect of the YT salt on the onset of disease in SOD1 G93A mutant ALS model mice. In the experiment, the model group did not receive any drug treatment, and the YT salt group was given the drug at a dose of 496 mg/kg (administered intragastrically once daily). Data analysis was performed using the Log-rank test, with P < 0.05 indicating a statistical difference between different groups.
FIG. 5 depicts the effect of the YT salt on the occurrence of severe conditions in SOD1 ^{G93A} mutant ALS model mice. In the experiment, the model group did not receive any drug treatment, and the YT salt group was given the drug at a dose of 496 mg/kg (administered intragastrically once daily).Data analysis was performed using the Log-rank test, with P < 0.05 indicating a statistical difference between different groups.

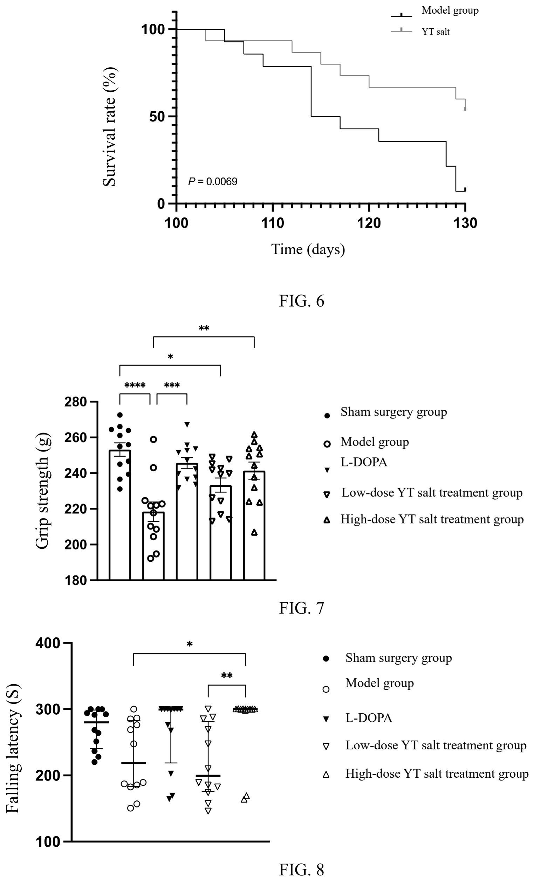

FIG. 6 depicts the effect of the YT salt on the survival period of SOD1^{G93A} mutant ALS model mice. In the experiment, the model group did not receive any drug treatment, and the YT salt group was given the drug at a dose of 496 mg/kg (administered intragastrically once daily). Data analysis was performed using the Log-rank test, with P < 0.05 indicating a statistical difference between different groups.
FIG. 7 depicts the effect of the YT salt on the grip strength of an MPTP-induced Parkinson's disease mouse model. In the experiment, the sham surgery group consisted of C57BL/6 mice intraperitoneally injected with 10 mL/kg normal saline; the model group consisted of C57BL/6 mice, which were intraperitoneally injected with 30 mg/kg MPTP for modeling and did not receive any drug treatment; the L-DOPA group served as the positive drug control group, in which model mice were treated with 40 mg/kg L-DOPA (administered intragastrically twice daily); the low-dose YT salt group consisted of model mice treated with 167.5 mg/kg YT salt (administered intragastrically twice daily); the high-dose YT salt group consisted of model mice treated with 248 mg/kg YT salt (administered intragastrically twice daily). The data in the figure are all expressed as mean ± standard error of the mean (SEM). P < 0.05 indicates statistical significance; * indicates P < 0.05, ** indicates P < 0.01, *** indicates P < 0.001, and **** indicates P < 0.0001.
FIG. 8 depicts the effect of the YT salt on the performance of an MPTP-induced Parkinson's disease mouse model in a rotarod test, which evaluates the time for animals to maintain balance on the rotating rod until falling to the ground. In the experiment, the sham surgery group consisted of C57BL/6 mice intraperitoneally injected with 10 mL/kg normal saline; the model group consisted of C57BL/6 mice, which were intraperitoneally injected with 30 mg/kg MPTP for modeling and did not receive any drug treatment; the L-DOPA group served as the positive drug control group, in which model mice were treated with 40 mg/kg L-DOPA (administered intragastrically twice daily); the low-dose YT salt group consisted of model mice treated with 167.5 mg/kg YT salt (administered intragastrically twice daily); the high-dose YT salt group consisted of model mice treated with 248 mg/kg YT salt (administered intragastrically twice daily). The data in the figure are all expressed as mean ± standard error of the mean (SEM). P < 0.05 indicates statistical significance; * indicates P < 0.05, and ** indicates P < 0.01.

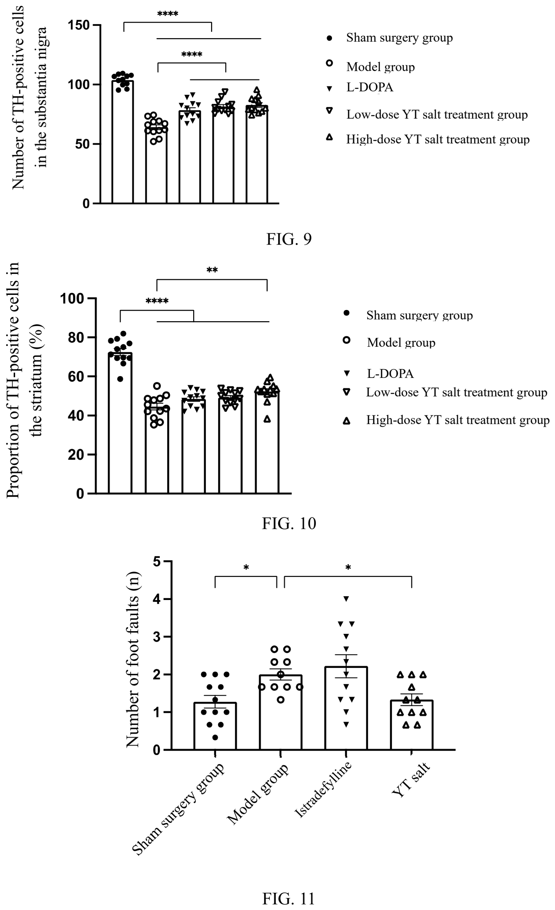

FIG. 9 depicts the effect of the YT salt on the number of TH-positive cells in the substantia nigra region of the MPTP-induced Parkinson's disease mouse model. In the experiment, the sham surgery group consisted of C57BL/6 mice intraperitoneally injected with 10 mL/kg normal saline; the model group consisted of C57BL/6 mice, which were intraperitoneally injected with 30 mg/kg MPTP for modeling and did not receive any drug treatment; the L-DOPA group served as the positive drug control group, in which model mice were treated with 40 mg/kg L-DOPA (administered intragastrically twice daily); the low-dose YT salt group consisted of model mice treated with 167.5 mg/kg YT salt (administered intragastrically twice daily); the high-dose YT salt group consisted of model mice treated with 248 mg/kg YT salt (administered intragastrically twice daily). The data in the figure are all expressed as mean ± standard error of the mean (SEM). P *<* 0.05 indicates statistical significance; **** indicates P < 0.0001.
FIG. 10 depicts the effect of the YT salt on the proportion of TH-positive cells in the striatal region of the brain of the MPTP-induced Parkinson's disease mouse model. In the experiment, the sham surgery group consisted of C57BL/6 mice intraperitoneally injected with 10 mL/kg normal saline; the model group consisted of C57BL/6 mice, which were intraperitoneally injected with 30 mg/kg MPTP for modeling and did not receive any drug treatment; the L-DOPA group served as the positive drug control group, in which model mice were treated with 40 mg/kg L-DOPA (administered intragastrically twice daily); the low-dose YT salt group consisted of model mice treated with 167.5 mg/kg YT salt (administered intragastrically twice daily); the high-dose YT salt group consisted of model mice treated with 248 mg/kg YT salt (administered intragastrically twice daily). The data in the figure are all expressed as mean ± standard error of the mean (SEM). P < 0.05 indicates statistical significance; ** indicates P < 0.01, and **** indicates P < 0.0001.
FIG. 11 depicts the effect of the YT salt on the number of foot faults in a grid walking test of 6-OHDA-induced Parkinson's disease rats. In the experiment, the sham surgery group consisted of SD rats without modeling treatment; the model group consisted of SD rats, which were injected with 4 µL of 6-OHDA (5 µg/µL) into the right intracranial medial forebrain bundle (MFB) for modeling and did not receive any drug treatment; the istradefylline group served as the positive drug control group, in which model rats were treated with 10 mg/kg istradefylline (administered intragastrically once daily); the YT salt treatment group consisted of model rats treated with 248 mg/kg YT salt (administered intragastrically once daily). The data in the figure are all expressed as mean ± standard error of the mean (SEM). P < 0.05 indicates statistical significance; * indicates *P* < 0.05.

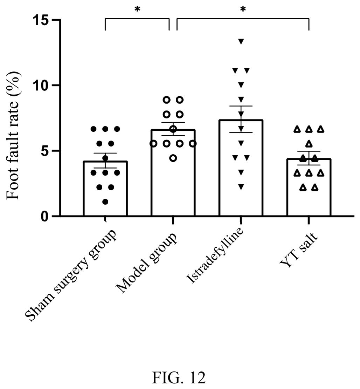

FIG. 12 depicts the effect of the YT salt on the foot fault rate in the grid walking test of 6-OHDA-induced Parkinson's disease rats. In the experiment, the sham surgery group consisted of SD rats injected with 4 µL of ascorbic acid solution (0.2 mg/mL) into the right intracranial medial forebrain bundle (MFB); the model group consisted of SD rats, which were injected with 4 µL of 6-OHDA (5 µg/µL) into the right intracranial MFB for modeling and did not receive any drug treatment; the istradefylline group served as the positive drug control group, in which model rats were treated with 10 mg/kg istradefylline (administered intragastrically once daily); the YT salt treatment group consisted of model rats treated with 248 mg/kg YT salt (administered intragastrically once daily). The data in the figure are all expressed as mean ± standard error of the mean (SEM). P < 0.05 indicates statistical significance; * indicates P < 0.05.

### DETAILED DESCRIPTION

### Definitions

In the present disclosure, unless otherwise stated, the scientific and technical terms used herein have the meanings generally understood by those skilled in the art. Moreover, the terms and laboratory procedures related to protein and nucleic acid chemistry, molecular biology, cell and tissue culture, microbiology, and immunology used herein are the terms and conventional procedures widely used in the corresponding fields. Meanwhile, in order to better understand the present disclosure, the definitions and explanations of the related terms are provided below.

As used herein, the term "halogen" refers to fluorine, chlorine, bromine, or iodine.

As used herein, the term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. Lower alkyl groups containing 1 to 6 carbon atoms are more preferred, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituent may be attached at any accessible point of attachment, and the substituent is preferably one or more groups independently selected from alkyl, alkenyl, alkynyl, alkoxy, alkylthio, alkylamino, halogen, sulfhydryl, hydroxyl, nitro, amino, cycloalkyl, heterocycloalkyl, aryl, heteroaryl, cycloalkoxy, heterocycloalkoxy, cycloalkylthio, heterocycloalkylthio, and oxo.

As used herein, the term "alkoxy" refers to -O-(alkyl) and -O-(cycloalkyl), wherein the alkyl or cycloalkyl is as defined above. Non-limiting examples of alkoxy include: methoxy, ethoxy, propoxy, butoxy, cyclopropyloxy, cyclobutyloxy, cyclopentyloxy, and cyclohexyloxy. Alkoxy may be optionally substituted or unsubstituted, and when it is substituted, the substituent is preferably one or more groups independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, and heterocycloalkylthio.

As used herein, the terms "halo" and "halogen" refer to fluorine, chlorine, bromine, and iodine.

As used herein, the term "haloalkyl", by itself or as part of another substituent, refers to an alkyl group in which some or all of the hydrogen atoms are replaced by halogen atoms. For alkyl groups, the haloalkyl may have any suitable number of carbon atoms, e.g., C1-6. For example, the haloalkyl includes trifluoromethyl, fluoromethyl, etc. In some contexts, the term "perfluoro" can be used to define a compound or group in which all hydrogen atoms are replaced by fluorine atoms. For example, perfluoromethyl refers to 1,1,1-trifluoromethyl.

As used herein, the term "hydroxyl" refers to an -OH moiety.

The present disclosure also comprises various deuterated forms of formula (I). Each available hydrogen atom attached to a carbon atom may be independently replaced by a deuterium atom. Those skilled in the art are able to synthesize the deuterated forms of formula (I) with reference to the relevant literature. Commercially available deuterated starting materials can be used in preparing the deuterated forms of formula (I), or they can be synthesized using conventional techniques with deuterated reagents. Non-limiting examples of deuterated reagents include: deuterated borane, tri-deuterated borane in tetrahydrofuran, deuterated lithium aluminum hydride, deuterated iodoethane, deuterated iodomethane, etc.

"Pharmaceutically acceptable" means that the component is compatible with the other components of the formulation and is harmless to the recipient.

As used herein, the term "pharmaceutically acceptable salt", when used in this context, refers to a pharmaceutically acceptable organic or inorganic salt of the compound. Exemplary salts include, but are not limited to, sulfate, citrate, acetate, oxalate, hydrochloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)). The pharmaceutically acceptable salt may involve the inclusion of another molecule, such as an acetate ion, a succinate ion, or another counterions. The counterion may be any organic or inorganic moiety that stabilizes the charge of the parent compound. In addition, the pharmaceutically acceptable salt may have more than one charged atom in the structure. In cases where multiple charged atoms are components of a pharmaceutically acceptable salt, multiple counterions can be present. Therefore, the pharmaceutically acceptable salt may have one or more charged atoms and/or one or more counterions.

As used herein, the term "acid anion" refers to an anion produced upon ionization of an acid. Common acid anions include chloride ion, bromide ion, iodide ion, carbonate ion, bicarbonate ion, sulfate ion, phosphate ion, hydrogen phosphate ion, dihydrogen phosphate ion, formate ion, acetate ion, etc. The valence of the acid anion may be -1, -2, -3, -4, etc.

As used herein, the term "pharmaceutically acceptable carrier" refers to a conventional non-toxic solid, semi-solid, or liquid filler, diluent, encapsulating material, formulation auxiliary, or carrier used in the art together with a therapeutic agent, collectively constituting a "pharmaceutical composition" for administration to an individual. Pharmaceutically acceptable carriers are non-toxic to recipients at the doses and concentrations employed and are compatible with the other ingredients of the formulation. Pharmaceutically acceptable carriers are suitable for the formulation employed.

As used herein, the term "pharmaceutically acceptable excipient" refers to a substance that facilitates the administration of an active agent to an individual. Useful pharmaceutical excipients include, but are not limited to, binders, fillers, disintegrants, lubricants, glidants, coating agents, sweetening agents, flavoring agents, and coloring agents.

Depending on the location and nature of the various substituents desired, the compounds of the present disclosure may contain one or more asymmetric centers. Asymmetric carbon atoms may exist in the (R)- and/or (S)-configuration, resulting in racemic mixtures in the case of a single asymmetric center, and in diastereomeric mixtures in the case of multiple asymmetric centers. In some cases, asymmetry may also exist due to hindered rotation about a particular bond, such as a central bond connecting two substituted aromatic rings of a particular compound. Substituents on rings may also exist in cis or trans forms. It is intended that all such configurations (including enantiomers and diastereomers) are included within the scope of the present disclosure. Preferred compounds are those that produce more of the desired biological activities. Separated, pure, or partially purified isomers and stereoisomers, or racemic or diastereomeric mixtures of the compounds of the present disclosure are all encompassed within the scope of the present disclosure. Purification and separation of such substances can be accomplished by standard techniques known in the art.

Tautomers, sometimes referred to as proton-shift tautomers, are two or more compounds that are associated by the migration of a hydrogen atom, accompanied by the switching of one or more single bonds and one or more adjacent double bonds. The compounds of the present disclosure may exist in one or more tautomeric forms.

The present disclosure also includes all suitable isotopic variants of the compounds of the present disclosure. Isotopic variants of the compounds of the present disclosure are defined as: compounds of the present disclosure in which at least one atom is replaced by an atom with the same atomic number but an atomic mass different from the atomic mass usually or predominantly found in nature. Examples of isotopes that can be incorporated into the compounds of the present disclosure include isotopes of hydrogen, carbon, nitrogen, oxygen, phosphorus, sulfur, fluorine, chlorine, bromine, and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I, and ¹³¹I. Certain isotopic variants of the compounds of the present disclosure, for example, those variants into which one or more radioactive isotopes (e.g., ³H or ¹⁴C) are incorporated, can be used in drug and/or substrate tissue distribution studies. Tritium and carbon-14 (i.e., ¹⁴C) isotopes are particularly preferred due to their ease of preparation and detectability. Furthermore, substitution with isotopes such as deuterium may afford certain therapeutic advantages resulting from better metabolic stability, for example, increased *in vivo* half-life or reduced dose requirements, and hence may be preferred in some circumstances. Isotopic variants of the compounds of the present disclosure can generally be prepared by employing conventional methods known to those skilled in the art, for example, by employing the illustrative methods or the preparation methods described in the examples below using appropriate isotopic variants of suitable reagents.

The present disclosure includes all possible stereoisomers of the compounds of the present disclosure as single stereoisomers or any mixture of said stereoisomers in any ratio. Separation of single stereoisomers, such as single enantiomers or single diastereomers, of the compounds of the present disclosure may be achieved by any suitable method in the prior art, such as chromatography, particularly, for example, chiral chromatography.

The present disclosure includes all possible tautomers of the compounds of the present disclosure, either as single tautomers or as any mixture of said tautomers in any ratio.

As used herein, the term "solvate" refers to a pharmaceutically acceptable solvate formed by the compound of the present disclosure with one or more solvent molecules; non-limiting examples of the solvent molecules include water, ethanol, acetonitrile, isopropanol, DMSO, ethyl acetate, etc. Hydrates are a specific form of solvates in which the solvent is water.

As used herein, the terms "effective amount" and "therapeutically effective amount" refer to a dose of a compound, such as the compound, the compound combination or the salt form of the present disclosure, that produces the therapeutic effect for which it is administered. The exact dose will depend on the purpose of the treatment and will be determined by those skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); Goodman & Gilman's The Pharmacological Basis of Therapeutics, 11th Edition, 2006, Brunton, Ed., McGraw-Hill; and Remington: The Science and Practice of Pharmacy, 21st Edition, 2005, Hendrickson, Ed., Lippincott, Williams & Wilkins).

As used herein, "treatment" or "treating" is an approach for obtaining beneficial or desired results (including clinical results). For the purposes of the present application, beneficial or desired clinical results include, but are not limited to, one or more of the following: alleviating one or more symptoms resulting from the disease, reducing the severity of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or complete) of the disease, decreasing the dose of one or more other medications required to treat the disease, delaying the progression of the disease, increasing or improving the quality of life, increasing weight gain, and/or extending survival. "Treatment" also encompasses reducing the pathological consequences (such as, for example, tumor volume) of cancer. The methods of the present application contemplate any one or more of these aspects of treatment.

As used herein, "prevention" or "preventing" includes providing prophylaxis against the occurrence or recurrence of a disease in an individual who may be predisposed to the disease but has not yet been diagnosed with the disease.

The terms "subject", "individual", and "patient" are used interchangeably herein to refer to a mammal, including, but not limited to, human, bovine, horse, feline, canine, rodent, or primate. In some embodiments, the individual is a human.

An "effective amount" of a pharmaceutical agent refers to an amount effective, at the required dose and over the required period of time, to achieve the desired therapeutic or prophylactic result. The specific dose may vary depending on one or more of the following: the particular pharmaceutical agent chosen, the dosing regimen to be followed, whether it is administered in combination with other compounds, the timing of administration, the tissue to be imaged, and the physical delivery system in which it is carried.

As used herein, the term "synergy" or "synergistic" is used to refer to a result of a combination of two compounds, components, or targeting agents that is greater than the sum of the individual pharmaceutical agents together. The term "synergy" or "synergistic" refers to an amelioration in a disease condition or disorder being treated as compared to the use of each compound, component, or targeting agent alone. This amelioration in the disease condition or disorder being treated is a "synergistic effect". A "synergistic amount" is an amount of a combination of two compounds, components, or targeting agents that results in a synergistic effect ("synergistic" is as defined herein).

The determination of the synergistic interaction between one or two components, the optimal range of the effect, and the absolute dose range of each component for the effect can be definitively measured by administering the components within different w/w ratio ranges and at different doses to patients in need of treatment. However, the observation of synergy in *in vitro* or *in vivo* models can be predictive of the effects in humans and other species, as well as in existing *in vitro* or *in vivo* models as described herein, for measuring synergistic effects. Furthermore, the results of such studies can also be used to predict the required effective dose and plasma concentration ratio ranges, as well as absolute doses and plasma concentrations, in humans and other species by applying pharmacokinetic/pharmacodynamic methods.

It should be understood that the embodiments of the application described herein include "consisting of..." and/or "consisting essentially of...".

Reference herein to an "about" value or parameter includes (and describes) variations that are directed to that value or parameter itself. For example, a description referring to "about X" includes a description of "X".

As used herein, the term "about X-Y" has the same meaning as "about X to about Y".

As used herein, the term "about", when used to modify the quantity of an ingredient or reactant of the present disclosure, refers to variations in the numerical amount that may occur, for example, from typical measurements and liquid handling procedures used in preparing concentrates or working solutions; from accidental errors in these procedures; from differences in the manufacture, source, or purity of the ingredients used in preparing the composition or implementing the method; etc. The term "about" also encompasses amounts that vary due to different equilibrium conditions relative to the composition resulting from a particular starting mixture. Whether modified by the term "about" or not, the claims include equivalents to the amounts. In one embodiment, the term "about" means a variation within 10% of the reported numerical value, preferably a variation within 5% of the reported numerical value.

It should be understood by those skilled in the art that when a numerical value is recited in a claim, whether it is prefixed with "about" or not, the actual value of the numerical value may vary above or below the recited value by 10% (± 10%), preferably by 5% (±5%).

When used herein and in the appended claims, the singular forms "a", "an", "or", and "the" include plural referents, unless the context clearly dictates otherwise.

### Description of Embodiments

### Combination Product

In some aspects, the present disclosure provides a combination product, which comprises:
(a) a compound of formula (I) or a pharmaceutically acceptable salt, a solvate, a hydrate, or a stereoisomer thereof: 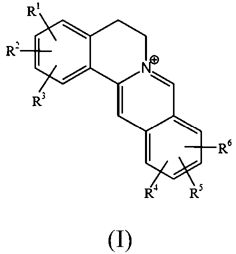
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N; and
(b) a bile acid or a derivative or an analog thereof, or a pharmaceutically acceptable salt, a solvate, a hydrate, or a stereoisomer thereof;
   wherein the molar ratio of the component (a) to the component (b) is 1:1000-1000:1.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate. Preferably, the pharmaceutically acceptable salt of the compound of formula (I) is selected from a maleate and a hydrochloride. More preferably, the pharmaceutically acceptable salt of the compound of formula (I) is a hydrochloride.

In a further embodiment, the compound of formula (I) is selected from: 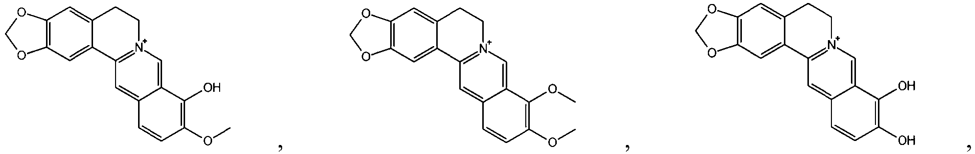 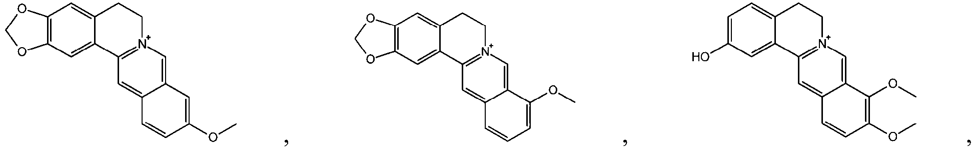 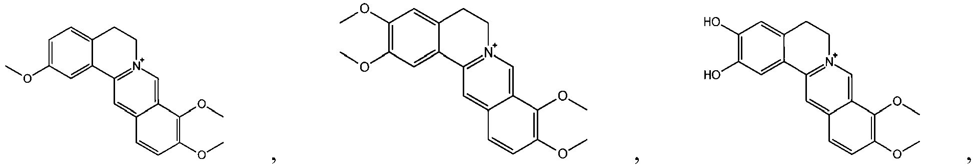 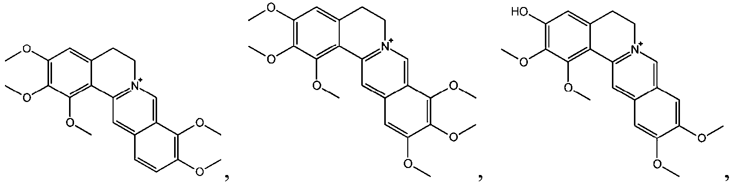 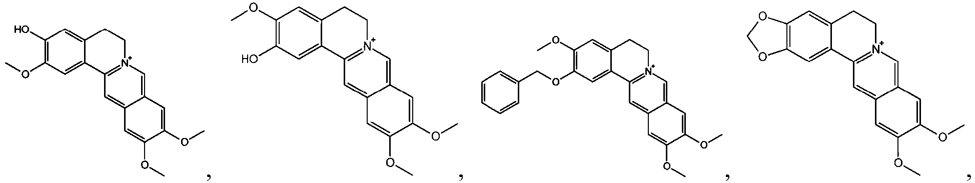 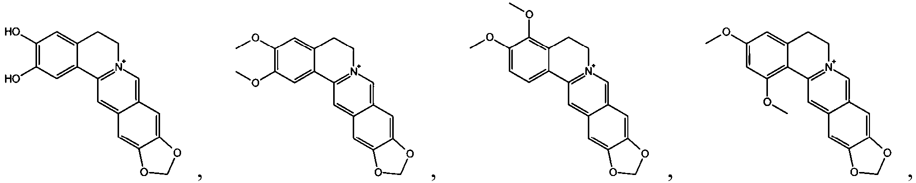 and

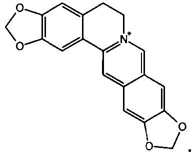

In a further embodiment, the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, the molar ratio of the component (a) to the component (b) is 1:100-100:1. Preferably, the molar ratio of the component (a) to the component (b) is 1:90-90:1, 1:80-80:1, 1:70-70:1, 1:60-60:1, 1:50-50:1, 1:40-40:1, 1:30-30:1, 1:20-20:1, or 1:10-10:1. More preferably, the molar ratio of the component (a) to the component (b) is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, or a numerical value between any two of these ratios.

In a further embodiment, the combination product is used for improving the viability of cells, particularly neuronal cells, especially by reducing active oxidative metabolite-mediated oxidative damage in the cells, modulating redox homeostasis in the cells, or reducing mitochondrial dysfunction in the cells. Preferably, the combination product is used for treating, alleviating, and/or preventing a neurodegenerative disease or disorder. More preferably, the neurodegenerative disease or disorder is selected from Alzheimer's disease (AD), Huntington's disease (HD), Parkinson's disease (PD), amyotrophic lateral sclerosis (ALS), sarcopenia, muscular dystrophy (MD), Pick's disease (PID), multi-infarct dementia (MID), Creutzfeldt-Jakob disease (CJD), dementia with Lewy bodies (DLB), mixed dementia, and frontotemporal dementia (FTD). Preferably, the neurodegenerative disease or disorder is selected from Alzheimer's disease (AD), Parkinson's disease (PD), and amyotrophic lateral sclerosis (ALS).

### Pharmaceutical Composition

In some aspects, the present disclosure provides a pharmaceutical composition, which comprises:
(a) a compound of formula (I) or a pharmaceutically acceptable salt, a solvate, a hydrate, or a stereoisomer thereof: 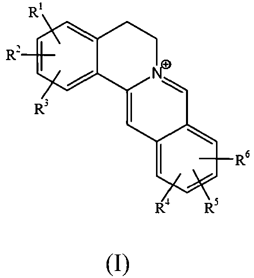
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N;
(b) a bile acid or a derivative or an analog thereof, or a pharmaceutically acceptable salt, a solvate, a hydrate, or a stereoisomer thereof; and
(c) a pharmaceutically acceptable carrier, excipient, and/or diluent;
   wherein the molar ratio of the component (a) to the component (b) is 1:1000-1000:1.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate. Preferably, the pharmaceutically acceptable salt of the compound of formula (I) is selected from a maleate and a hydrochloride. More preferably, the pharmaceutically acceptable salt of the compound of formula (I) is a hydrochloride.

In a further embodiment, the compound of formula (I) is selected from: 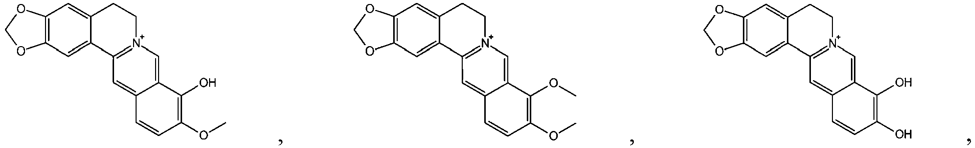 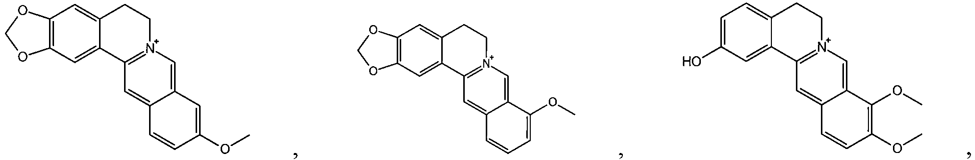 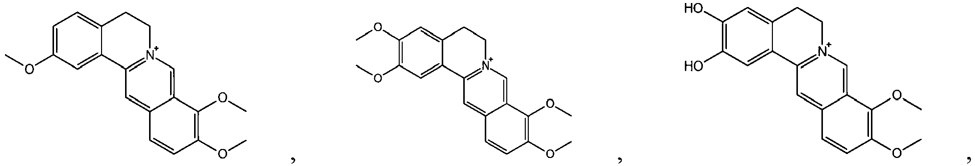 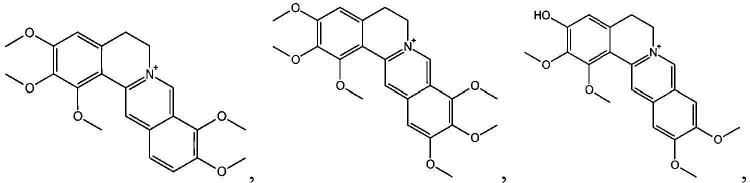 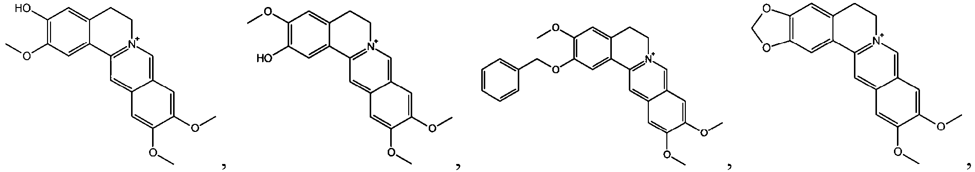 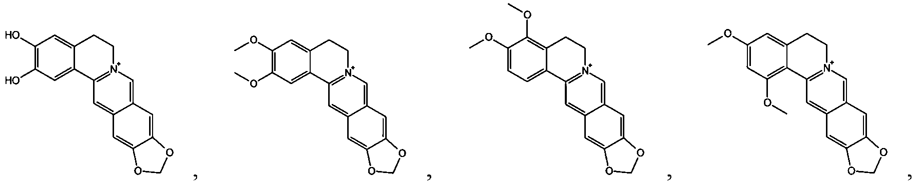 and

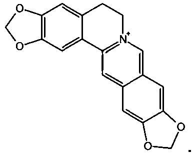

In a further embodiment, the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, the molar ratio of the component (a) to the component (b) is 1:100-100:1. Preferably, the molar ratio of the component (a) to the component (b) is 1:90-90:1, 1:80-80:1, 1:70-70:1, 1:60-60:1, 1:50-50:1, 1:40-40:1, 1:30-30:1, 1:20-20:1, or 1:10-10:1. More preferably, the molar ratio of the component (a) to the component (b) is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, or a numerical value between any two of these ratios.

In a further embodiment, the pharmaceutical composition is used for improving the viability of cells, particularly neuronal cells, especially by reducing active oxidative metabolite-mediated oxidative damage in the cells, modulating redox homeostasis in the cells, or reducing mitochondrial dysfunction in the cells. Preferably, the pharmaceutical composition is used for treating, alleviating, and/or preventing a neurodegenerative disease or disorder. More preferably, the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia. Preferably, the neurodegenerative disease or disorder is selected from Alzheimer's disease (AD), Parkinson's disease (PD), and amyotrophic lateral sclerosis (ALS).

### Acid-Base Addition Salts

In some aspects, the present disclosure provides an acid-base addition salt of formula (II):

(A⁺)ₘ(B⁻)ₙ(C⁻)ₚ (II)

wherein
(a) A⁺ is a cationic moiety, which is a compound of formula (I): 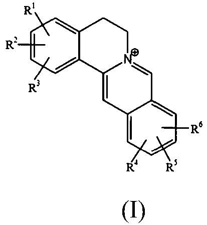
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N;
(b) B⁻ is an anionic moiety, which is a bile acid or a derivative or an analog thereof; and
(c) C⁻ is an acid anion;
   wherein m, n, and p are each independently an integer selected from 1-6, such that the configuration of the salt reaches charge balance, and when m = n, p is 0.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the compound of formula (I) is a cationic moiety selected from the group consisting of: 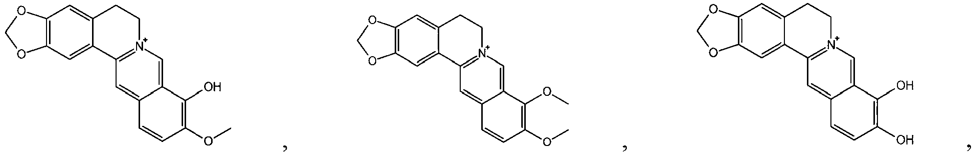 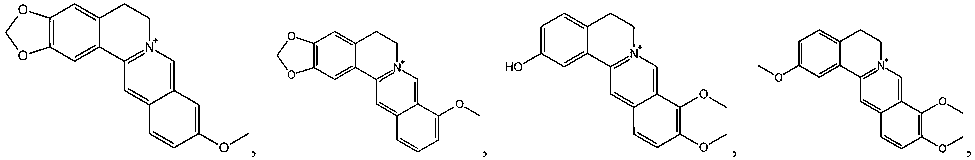 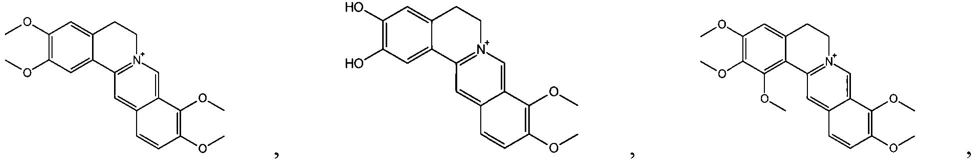 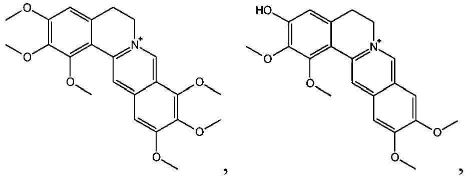 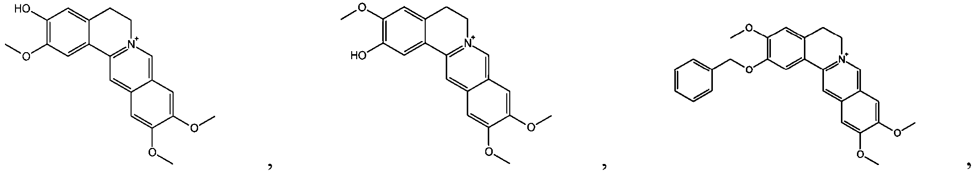 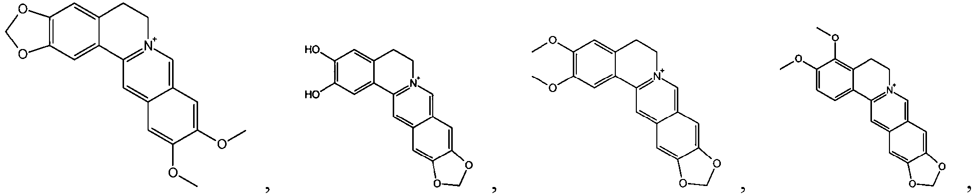 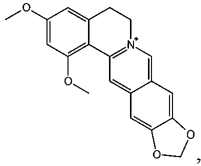 and

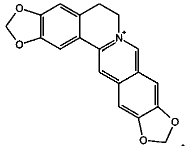

In a further embodiment, the component (b) is an anionic moiety selected from the group consisting of: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, C⁻ is a monovalent, divalent, trivalent, or tetravalent acid anion, or a mixture thereof. Preferably, C⁻ is a monovalent, divalent, or trivalent acid anion, or a mixture thereof. More preferably, C⁻ is a monovalent or divalent acid anion, or a mixture thereof. Most preferably, C⁻ is a monovalent acid anion or a mixture thereof.

In a further embodiment, m is 1, n is 1, and p is 0; or m is 2, n is 1, and p is 1.

In a further embodiment, the pharmaceutical composition is used for improving the viability of cells, particularly neuronal cells, especially by reducing active oxidative metabolite-mediated oxidative damage in the cells, modulating redox homeostasis in the cells, or reducing mitochondrial dysfunction in the cells. Preferably, the pharmaceutical composition is used for treating, alleviating, and/or preventing a neurodegenerative disease or disorder. More preferably, the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia. Preferably, the neurodegenerative disease or disorder is selected from Alzheimer's disease (AD), Parkinson's disease (PD), and amyotrophic lateral sclerosis (ALS).

### Treatment and/or Prevention

In some aspects, the present disclosure provides a method for treating, alleviating, and/or preventing a neurodegenerative disease or disorder. In some aspects, the method for treating, alleviating, and/or preventing the neurodegenerative disease or disorder comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing Alzheimer's disease (AD), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing Huntington's disease (HD), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing Parkinson's disease (PD), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing amyotrophic lateral sclerosis (ALS), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing Pick's disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing multi-infarct dementia, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing Creutzfeldt-Jakob disease, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing dementia with Lewy bodies (DLB), which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing mixed dementia, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating, alleviating, and/or preventing frontotemporal dementia, which comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a method for treating and/or preventing a neurodegenerative disease or disorder, which comprises: regimen 1: administering to a patient in need thereof:
(a) a therapeutically and/or prophylactically effective amount of a compound of formula (I) or a pharmaceutically acceptable salt, a solvate, a hydrate, or a stereoisomer thereof: 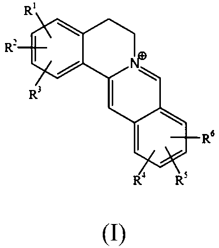
   wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
   or any two adjacent ones of R¹, R², and R³, together with the carbon atoms to which they are attached, form a 4- to 7-membered heterocyclic ring containing one or more heteroatoms selected from O, S, and N;
   or any two adjacent ones of R⁴, R⁵, and R⁶, together with the carbon atoms to which they are attached, form a 4- to 7-membered heterocyclic ring containing one or more heteroatoms selected from O, S, and N; or
   regimen 2: administering to a patient in need thereof the following components simultaneously, concurrently, separately, or sequentially:
      (a) a therapeutically and/or prophylactically effective amount of a compound of formula (I) or a pharmaceutically acceptable salt, a solvate, a hydrate, or a stereoisomer thereof: 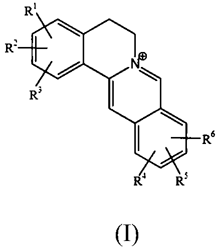
         wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
         or any two adjacent ones of R¹, R², and R³, together with the carbon atoms to which they are attached, form a 4- to 7-membered heterocyclic ring containing one or more heteroatoms selected from O, S, and N;
         or any two adjacent ones of R⁴, R⁵, and R⁶, together with the carbon atoms to which they are attached, form a 4- to 7-membered heterocyclic ring containing one or more heteroatoms selected from O, S, and N; and
      (b) a therapeutically and/or prophylactically effective amount of a bile acid or a derivative or an analog thereof, or a pharmaceutically acceptable salt, a solvate, a hydrate, or a stereoisomer thereof.

In a further embodiment, R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms. In a yet further embodiment, one or two of R¹, R², and R³ are hydrogen; or any two adjacent ones of R¹, R², and R³, together with the carbon atoms to which they are attached, form a 5-membered heterocyclic ring containing one or more oxygen atoms. In a still further embodiment, one of R¹, R², and R³ is hydrogen; or any two adjacent ones of R¹, R², and R³, together with the carbon atoms to which they are attached, form a 5-membered heterocyclic ring containing two oxygen atoms.

In a further embodiment, R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms. In a yet further embodiment, one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms. In a still further embodiment, one of R⁴, R⁵, and R⁶ is hydrogen; or any two adjacent ones of R⁴, R⁵, and R⁶, together with the carbon atoms to which they are attached, form a 5-membered heterocyclic ring containing two oxygen atoms.

In a further embodiment, the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate. Preferably, the pharmaceutically acceptable salt of the compound of formula (I) is selected from a maleate and a hydrochloride. More preferably, the pharmaceutically acceptable salt of the compound of formula (I) is a hydrochloride.

In a further embodiment, the compound of formula (I) is selected from: 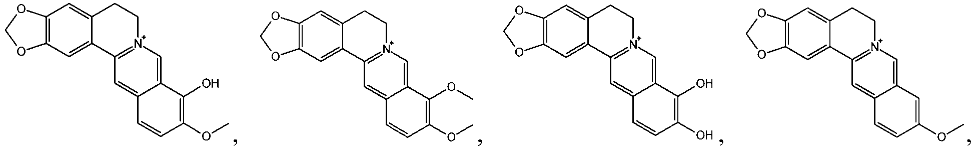 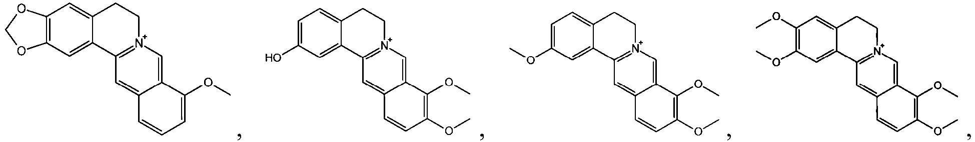 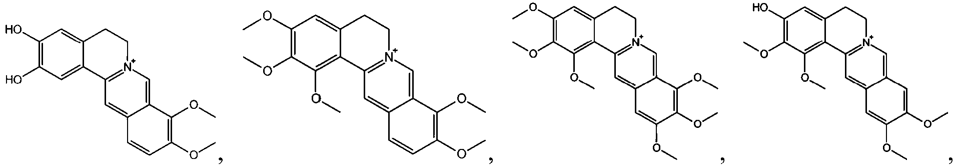 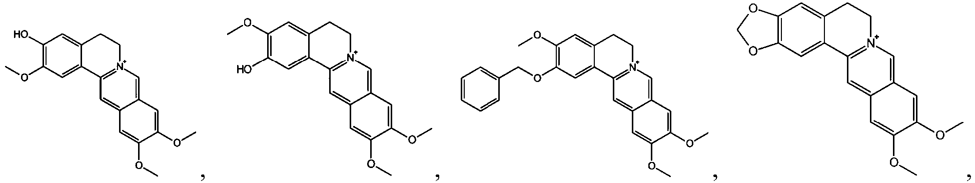 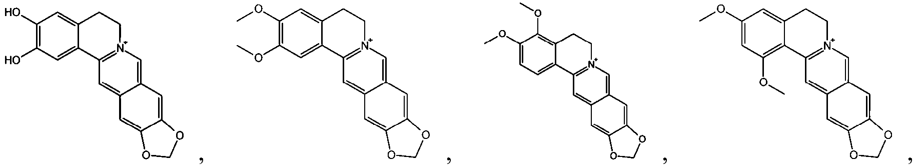 and

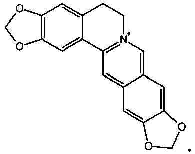

In a further embodiment, the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, tauroursodeoxycholic acid, etc.

In a further embodiment, the molar ratio of the component (a) to the component (b) is 1:100-100:1. Preferably, the molar ratio of the component (a) to the component (b) is 1:90-90:1, 1:80-80:1, 1:70-70:1, 1:60-60:1, 1:50-50:1, 1:40-40:1, 1:30-30:1, 1:20-20:1, or 1:10-10:1. More preferably, the molar ratio of the component (a) to the component (b) is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, or a numerical value between any two of these ratios.

In a further embodiment, the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia. Preferably, the neurodegenerative disease or disorder is selected from Alzheimer's disease (AD), Parkinson's disease (PD), and amyotrophic lateral sclerosis (ALS).

In some aspects, the present disclosure provides a method for improving the viability of cells, particularly neuronal cells, especially by reducing active oxidative metabolite-mediated oxidative damage in the cells, modulating redox homeostasis in the cells, or reducing mitochondrial dysfunction in the cells, which comprises contacting the cells with the combination product, the pharmaceutical composition, the acid-base addition salt, or the compound of formula (I) or the pharmaceutically acceptable salt, the solvate, the hydrate, or the stereoisomer thereof according to any one of the preceding embodiments.

In some aspects, the present disclosure provides a compound of formula (I) or a pharmaceutically acceptable salt, a solvate, a hydrate, or a stereoisomer thereof for use in improving the viability of cells, particularly neuronal cells, especially by reducing active oxidative metabolite-mediated oxidative damage in the cells, modulating redox homeostasis in the cells, or reducing mitochondrial dysfunction in the cells.

In some aspects, the present disclosure provides a compound of formula (I) or a pharmaceutically acceptable salt, a solvate, a hydrate, or a stereoisomer thereof for use in treating, alleviating, and/or preventing a neurodegenerative disease or disorder.

In a further embodiment, the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

### Mode of Administration

Administration of the compounds or salt forms in the combination of the present disclosure may be affected by any method capable of delivering the compound to the site of action. These methods include oral administration, intraduodenal administration, parenteral injection (including intravenous, subcutaneous, intramuscular, intravascular, or infusion), topical administration, and rectal administration.

The compounds or salt forms in the method or combination of the present disclosure may be formulated prior to administration. Preferably, the formulation will be suitable for the specific mode of administration. These compounds may be formulated with pharmaceutically acceptable carriers known in the art and administered in a variety of dosage forms known in the art. In the preparation of the pharmaceutical composition of the present disclosure, the active ingredient is typically mixed with a pharmaceutically acceptable carrier, or diluted with a carrier, or encapsulated within a carrier. Such carriers include, but are not limited to, solid diluents or fillers, excipients, sterile aqueous media, and various non-toxic organic solvents. Dosage unit forms or pharmaceutical compositions include tablets, capsules such as gelatin capsules, pills, powders, granules, aqueous and non-aqueous oral solutions and suspensions, lozenges, troches, hard candies, sprays, creams, salves, suppositories, pectins, gels, pastes, lotions, ointments, injectable solutions, elixirs, syrups, and parenteral solutions packaged in containers suitable for subdivision into individual doses.

Parenteral formulations include pharmaceutically acceptable aqueous or non-aqueous solutions, dispersions, suspensions, and emulsions, and sterile powders (for preparation thereof). Examples of carriers include water, ethanol, polyols (propylene glycol and polyethylene glycol), vegetable oils, and injectable organic esters such as ethyl oleate. Flowability may be maintained by using coatings such as lecithin or surfactants, or by maintaining an appropriate particle size. Exemplary parenteral administration forms include solutions or suspensions of the compounds of the present disclosure in sterile aqueous solutions such as aqueous propylene glycol solution or dextrose solution. Such dosage forms may be appropriately buffered, if necessary.

In addition, lubricants, such as magnesium stearate, sodium lauryl sulfate, and talc, are generally useful for tableting purposes. Similar types of solid compositions may also be used in soft and hard-filled gelatin capsules. Therefore, preferred materials include lactose (or milk sugar) and high molecular weight polyethylene glycols. When aqueous suspensions or elixirs are required for oral administration, the active compound therein may be combined with various sweetening or flavoring agents, coloring agents or dyes, and if necessary, emulsifying or suspending agents, as well as diluents such as water, ethanol, propylene glycol, glycerin, or combinations thereof.

Methods for preparing various pharmaceutical compositions using specific amounts of the active compound are known or apparent to those skilled in the art. For example, see Remington's Pharmaceutical Sciences, Mack Publishing Company, Easter, Pa., 15th Edition (1975).

### Kit

In some aspects, the present disclosure provides a kit for treating, alleviating, and/or preventing a neurodegenerative disease or disorder, which comprises the combination product, the acid-base addition salt, the pharmaceutical composition, or the compound of formula (I) or the pharmaceutically acceptable salt, the solvate, the hydrate, or the stereoisomer thereof according to any one of the preceding embodiments, and instructions for use in administering the therapeutic agent. In one embodiment, the instructions for use detail and define the mode of administration of the therapeutic agents, e.g., for simultaneous, concurrent, separate, or sequential administration of the therapeutic agents of the present disclosure. In one embodiment, the instructions for use detail and define the mode of administration of the therapeutic agents, for example, by specifying the days of administration for each therapeutic agent during a specific period of time.

In some aspects, the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia. Preferably, the neurodegenerative disease or disorder is selected from Alzheimer's disease (AD), Parkinson's disease (PD), and amyotrophic lateral sclerosis (ALS).

### Examples

The present disclosure will be further described in detail with reference to the following examples, which should not be construed as limiting the present disclosure.

**Materials and reagents:** Unless otherwise stated, all reagents involved in the experimental section of the present disclosure are commercially available products. For example, berberine hydrochloride (compound B), ursodeoxycholic acid (compound U), and tauroursodeoxycholic acid (compound T) were purchased from Energy Chemical. Neuroblastoma SH-sy5y cells were purchased from the Cell Bank of the Chinese Academy of Sciences.

In obtaining the compounds and the corresponding analytical data described in the examples below, the following experimental and analytical protocols were followed, unless otherwise indicated.

LC-MS: Unless otherwise stated, the analytical LC-MS system used consisted of a Shimadzu LCMS-2020 with electrospray ionization (ESI) in positive ion detection mode, equipped with a 20ADXR pump, an SIL-20ACXR autosampler, a CTO-20AC column oven, an M20APDA detector, and an LCMS 2020MS detector. The column was HALO, specifically C18 30 mm × 5.0 mm, 2.7 µm. Mobile phase A was water containing 0.05% TFA, and mobile phase B was acetonitrile containing 0.05% TFA. The gradient was set as follows: changed from 5% mobile phase B to 100% (95%) in 2.0 min, maintained for 0.7 min, then returned to 5% mobile phase B in 0.05 min and maintained for 0.25 min. The column oven (CTO-20AC) was operated at 40.0 °C. The flow rate was 1.5 mL/min, and the injection volume was 1 µL. The PDA (SPD-M20A) detection range was from 190 nm to 400 nm. MS detector, configured with electrospray ionization as the ionizable source; acquisition mode: scanning; nebulizing gas flow rate: 1.5 L/min; drying gas flow rate: 15 L/min; detector voltage: tuned voltage ± 0.2 kV; DL temperature: 250 °C; heating block temperature: 250 °C; scanning range: 90.00 m/z to 900.00 m/z. ELSD (Alltech 3300) detector parameters: drift tube temperature: 60 ± 5 °C; N2 flow rate: 1.8 ± 0.2 L/min. The mobile phase gradient was optimized for each compound. The calculated mass corresponds to the exact mass.

Preparative HPLC: Unless otherwise stated, preparative HPLC purification was performed using a Waters Auto purification system (2545-2767) equipped with a 2489 UV detector. The column was selected from one of the following: Waters C18, 19 mm × 150 mm, 5 µm; XBridge Prep OBD C18 column, 30 mm × 150 mm, 5 µm; XSelect CSH Prep C18 OBD column, 5 µm, 19 mm × 150 mm; XBridge Shield RP18 OBD column, 30 mm × 150 mm, 5 µm; XSelect CSH Fluoro-Phenyl, 30 mm × 150 mm, 5 µm; or YMC-Actus Triart C18, 30 mm × 150 mm, 5 µm. The mobile phase consisted of a mixture of acetonitrile (5%-95%) in an aqueous solution containing 0.1% FA or 10 mmol/L NH₄HCO₃. The flow rate was maintained at 25 mL/min, the injection volume was 1200 µL, and the UV detector used two channels: 254 nm and 220 nm. The mobile phase gradient was optimized for each compound.

Chiral chromatography: Chiral analytical chromatography was performed on one of the following: Chiralpak AS, AD; Chiralcel OD, OJ; Chiralpak IA, IB, IC, ID, IE, IF, IG, IH columns (Daicel Chemical Industries, Ltd.); (R,R)-Whelk-Ol, (S,S)-Whelk-O1 columns (Regis technologies, Inc.); CHIRAL Cellulose-SB, SC, SA columns (YMC Co., Ltd.) as described; with different column dimensions (50 mm × 4.6 mm, 100 mm × 4.6 mm, 150 mm × 4.6 mm, 250 mm × 4.6 mm, 50 mm × 3.0 mm, 100 mm × 3.0 mm); using the percentage of ethanol in hexane (%Et/Hex) or isopropanol in hexane (%IPA/Hex) as an isocratic solvent system, or under supercritical fluid chromatography (SFC) conditions.

Normal phase flash chromatography: Unless otherwise stated, normal phase flash column chromatography (FCC) was performed on silica gel using a pre-packed silica gel column, with ethyl acetate (EtOAc)/hexane, ethyl acetate (EtOAc)/petroleum ether (b.p. 60-90 °C), CH₂Cl₂/MeOH, or CH₂Cl₂/10% 2 N NH₃ in MeOH as eluents.

¹H NMR: Unless otherwise stated, ¹H NMR spectra were obtained using a 400 MHz spectrometer (or a 300 MHz spectrometer) in a DMSO-d₆ solution. Nuclear magnetic resonance (NMR) spectroscopic characteristics refer to chemical shifts (δ) expressed in parts per million (ppm). Tetramethylsilane (TMS) was used as an internal standard in a DMSO-d₆ solution, and the residual CH₃OH peak or TMS was used as an internal standard in a CD₃OD solution. Coupling constants (J) are reported in Hertz (Hz). The nature of the shifts with respect to multiplicity is reported as s (singlet), d (doublet), t (triplet), q (quartet), dd (doublet of doublets), dt (doublet of triplets), m (multiplet), and br (broad).

Abbreviations used in this specification, particularly in the examples, are listed in the table below:

| | | | |
|---|---|---|---|
| THF | = | Tetrahydrofuran | |
| LAH | = | Lithium aluminum hydride | |
| Compound B | = | Berberine hydrochloride | 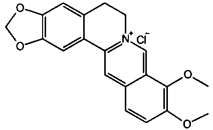 |
| Compound Y | = | 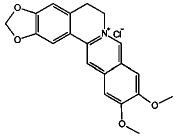 | |
| Compound U | = | Ursodeoxycholic acid | 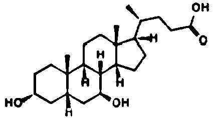 |
| Compound N | = | 24-Norursodeoxycholic acid | 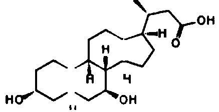 |
| Compound T | = | Tauroursodeoxycholic acid | 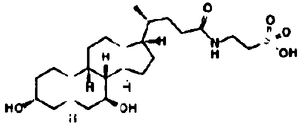 |
| DCM | = | Dichloromethane | |
| DCE | = | Dichloroethane | |
| MTBE | = | Methyl tert-butyl ether | |
| TFA | = | Trifluoroacetic acid | |
| YT salt | = | 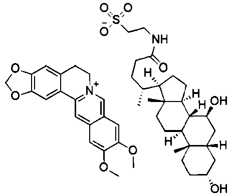 | |
| PB | = | Sodium phenylbutyrate | 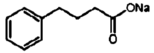 |

### Example 1. Synthesis of Compound Y

### Step 1. Synthesis of 2-(benzo[d][1,3]dioxol-5-yl)ethan-1-ol

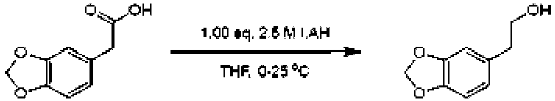

LAH (2.5 M, 1.00 L) was added dropwise to a solution of 1,3-benzodioxole-5-acetic acid (500 g, 2.78 mol) in THF (200 mL) at 0 °C, and then the mixture was stirred at 25 °C for 12 h under a N₂ atmosphere. The mixture was cooled to -10 to 0 °C, and then H₂O (95.0 mL), NaOH (15%, 95.0 mL), and H₂O (285 mL) were added sequentially. After 0.5 h, Na₂SO₄ (500 g) was added to the mixture, and the resulting mixture was stirred at 25 °C for 0.5 h. The mixture was filtered, and the filter cake was washed with THF (5.00 L). The filtrate was collected and concentrated to give the title compound (460 g, 2.77 mol, 99.7% yield) as a yellow oil.

### Step 2. Synthesis of 2-(benzo[d][1,3]dioxol-5-yl)ethane-1-pivalate

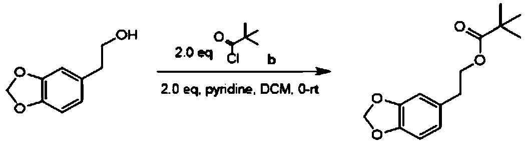

2-(Benzo[d][1,3]dioxol-5-yl)ethan-1-ol (455 g) and pyridine (435 g) were dissolved in DCM (2.00 L), and compound b (664 g) was added dropwise at 0 °C under a N₂ atmosphere. The mixture was stirred for 8 h. The residue was poured into water, and the aqueous phase was extracted with DCM. The organic phases were combined, washed with brine, dried, and concentrated. The residue was purified by silica gel chromatography to give the title compound (670 g) as a yellow oil.

HNMR (CDCl₃): 66.78-6.70 (m, 2H), 6.68-6.64 (m, 1H), 6.10-5.58 (m, 2H), 4.22 (t, J = 6.94 Hz, 2H), 2.84 (t, J = 6.94 Hz, 2H), 1.20-1.16 (m, 9H).

### Step 3. Synthesis of 2-(6-acetyl-benzo[d][1,3]dioxol-5-yl)ethane-1-pivalate

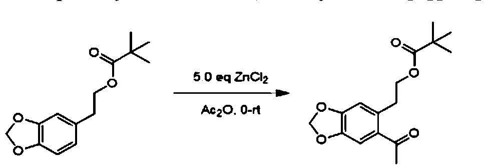

ZnCl₂ (1.01 kg) was added in one portion to a solution of 2-(benzo[d][1,3]dioxol-5-yl)ethane-1-pivalate (620 g) in Ac₂O (3.00 L) at 0 °C under N₂, and then the mixture was stirred at room temperature for 8 h. The reaction mixture was poured into water and then extracted with ethyl acetate. The organic phases were combined, washed with brine, dried, and concentrated. Purification was performed by silica gel chromatography to give the title compound (467 g) as a yellow oil.

HNMR (CDCl₃): *δ*7.23 (s, 1H), 6.75 (s, 1H), 6.78-6.70 (m, 1H), 6.02 (s, 2H), 4.26 (t, J = 6.63 Hz, 1H), 4.35-4.21(m, 1H), 3.17 (t, J = 6.63 Hz, 2H), 2.54 (s, 3H), 1.16 (s, 9H).

### Step 4. Synthesis of 2-(6-(2-(2-(1,3-dioxolan-2-yl)-4,5-dimethoxyphenyl)acetyl)benzo[d][1,3]dioxol-5-yl)ethyl pivalate

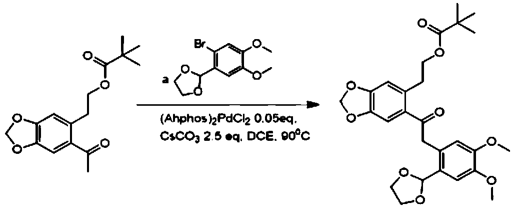

Cs₂CO₃ (451 g) and (Ahphos)₂PdCl₂ (58.8 g) were added in one portion to a solution of 2-(6-acetyl-benzo[d][1,3]dioxol-5-yl)ethane-1-pivalate (284 g) and compound a (200 g) in DCE at room temperature under a N₂ atmosphere. The mixture was left to react for 10 h with the temperature maintained at 90 °C. After the reaction was completed, the reaction system was slowly added dropwise to water and extracted with ethyl acetate. The organic phase was washed with brine, dried, and concentrated. Purification was performed by silica gel chromatography to give the title compound (310 g) as a yellow solid.

### Step 5. Synthesis of compound Y

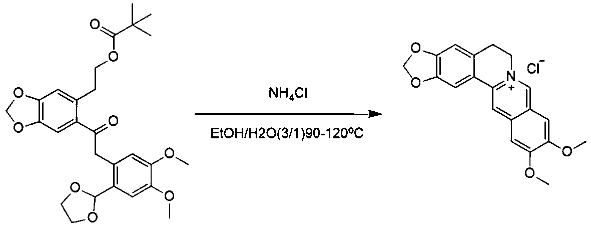

An NH₄Cl solution (3 M, 3.38 eq) was added in one portion to a solution of 2-(6-(2-(2-(1,3-dioxolan-2-yl)-4,5-dimethoxyphenyl)acetyl)benzo[d][1,3]dioxol-5-yl)ethyl pivalate (310 g) in EtOH at room temperature under a N₂ atmosphere, and then the mixture was left to react under high pressure at 120 °C for 24 h. After the reaction was completed, the mixture was cooled to 25 °C and filtered, and the filter cake was washed with MTBE. Purification was performed by silica gel chromatography to give the title compound (190 g).

HNMR (DMSO-*d*₆): *δ*9.73-9.41 (s, 1H), 8.91-8.58 (s, 1H), 7.79-7.66 (s, 2H), 7.62-7.55 (s, 1H), 7.15-7.04 (s, 1H), 6.23-6.09 (s, 2H), 4.86-4.69 (m, 2H), 4.10-4.04 (m, 3H), 4.02-3.97 (s, 3H), 3.25-3.15 (m, 2H).

### Example 2. Preparation and Characterization of YT Salt

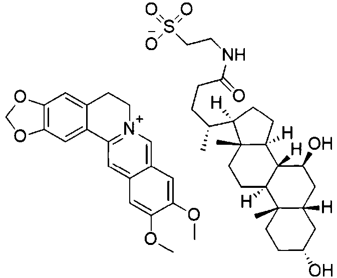

**Preparation of YT salt:** Compound Y and compound T were suspended in a mixed solvent of methanol/ethyl acetate (isopropyl acetate) (v/v: 1/9) and continuously stirred for 24 h, with a significant amount of solid suspended in the system. The solid was obtained by filtration and dried to give the YT salt form.

**Characterization of YT salt:** The ion ratio of Y to T in the YT salt was 1:1.

HNMR (DMSO-*d*₆):*δ*59.60(s, 1H), 8.77(s, 1H), 7.73~7.71(d, 1H), 7.70~7.69(t, 2H), 7.60(s, 1H), 7.09(s, 1H), 6.17(s, 2H), 4.79~4.77(t, 2H), 4.47~4.46(d, 1H), 4.07(s, 3H), 4.05~4.01(q, 1H), 4.00(s, 3H), 3.89~3.88(d, 1H), 3.31~3.25(m, 4H), 3.21~3.18(t, 2H), 2.55~2.53(t, 2H), 2.07~2.02(m, 1H), 1.99(s, 1H), 1.94~1.89(m, 2H), 1.85~1.80(m, 1H), 1.75~1.71(m, 1H), 1.68~1.60(m, 3H), 1.49~1.44(m, 3H), 1.42~1.26(m, 7H), 1.22~1.06(m, 7H), 1.00~0.96(q, 1H), 0.93~0.88(dt, 1H), 0.88~0.86(m, 6H), 0.60(s, 3H).

**Example 3. Effects of Compound Y Monotherapy (Y), Compound T Monotherapy (T), Compound B Monotherapy (B), Compound N Monotherapy (N), Compound Combination Y + T, Compound Combination B + N, Compound Combination PB + T, and Novel Salt YT on Cell Viability of Hydrogen Peroxide-Induced SH-sy5y Cell Line**

Neuroblastoma SH-sy5y cells (purchased from the Cell Bank of the Chinese Academy of Sciences) were cultured in a complete medium (DMEM + 10% FBS + 1% PS) in a cell incubator at 37 °C with 5% CO₂. The experiment was divided into 11 groups in total:
Group 1 (control): SH-sy5y cells
Group 2 (model): SH-sy5y cells + hydrogen peroxide
Group 3: SH-sy5y cells + hydrogen peroxide + compound Y (100 µM)
Group 4: SH-sy5y cells + hydrogen peroxide + compound T (100 µM)
Group 5: SH-sy5y cells + hydrogen peroxide + compound B (100 µM)
Group 6: SH-sy5y cells + hydrogen peroxide + compound N (100 µM)
Group 7: SH-sy5y cells + hydrogen peroxide + compound combination Y + T (100 µM + 100 µM)
Group 8: SH-sy5y cells + hydrogen peroxide + compound combination B + N (100 µM + 100 µM)
Group 9: SH-sy5y cells + hydrogen peroxide + compound combination PB + T (500 µM + 200 µM)
Group 10: SH-sy5y cells + hydrogen peroxide + YT salt (100 µM)
Group 11: SH-sy5y cells + DMSO

Neuroblastoma SH-sy5y cells were plated at 1 × 10⁴ cells/well in a complete medium (DMEM + 10% FBS + 1% PS) and cultured in a cell incubator at 37 °C with 5% CO₂ for 24 h, followed by the addition of the corresponding compounds. After 24 h of incubation, hydrogen peroxide was added to stimulate the cells for 1 h, with the final concentration of hydrogen peroxide being 350 µM. Subsequently, 50 µM CellTiter-Glo^{®} (CTG) reagent was added, and the mixture was shaken for 2 min. Then, the luminescence signal values were measured, which could characterize the cell viability.

As shown in FIG. 1, compared to the model (column 2), the compound Y monotherapy (column 3) exhibited an excellent effect in improving cell viability (P < 0.0001), and the effect of the compound Y monotherapy (column 3) was superior to that of the compound B monotherapy (column 5); compared to the compound T monotherapy (column 4), the compound B monotherapy (column 5), the compound combination B + N (column 8), and the compound combination PB + T (column 9), the compound combination Y + T (column 7) and the YT salt (column 10) exhibited significantly superior effects in improving cell viability, indicating that the compound combination Y + T and the YT salt were more effective in repairing oxidative stress-induced damage in neural cells than the compound T monotherapy, the compound B monotherapy, the compound combination B + N, and the compound combination PB + T. The compound combination PB + T was based on the concentrations of compound PB and compound T as documented in the data of the marketed drug AMX0035 (DavidL. Carbone. Pharmacology/toxicologyNDAreviewandevaluation, Application number: 216660Orig1s000, Food and Drug Administration. doi: pink.pharmaintelligence.informa.com/-/media/supporting-documents
/pink-sheet/2022/11/relyvrio_nonclinical.pdf?rev=d27109bf35c040d4a78f73144d132917&hash=414B B5B4473A21ECE0231375EB1C0341).

The above results show that the compound Y monotherapy, the compound combination Y + T, and the YT salt exhibited significantly superior effects in improving cell viability and repairing oxidative stress-induced damage in nerve cells, indicating their potential as drugs for treating, alleviating, and/or preventing neurodegenerative diseases or disorders.

### Example 4. Inhibitory Effects of Compound Y Monotherapy, Compound T Monotherapy, Compound Combination Y + T, and YT Salt on Proliferation of CD4-Positive and CD8-Positive T Cells

It has been reported that in ALS, Th cells are described as major players in inflammation and disease progression. In the 1990s, CD4 T cell infiltration was observed in the spinal cord near degenerative areas of ALS patients. Similarly, CD8 T cell infiltration was also observed in the spinal cord and brain of ALS patients. (Elise Liu, et al., Neuroinflammation in Amyotrophic Lateral Sclerosis and Frontotemporal Dementia and the Interest of Induced Pluripotent Stem Cells to Study Immune Cells Interactions With Neurons, Front Mol Neurosci. 2021 Dec 14; 14:767041. doi: 10.3389/fnmo1.2021.767041.) To observe the inhibitory effects of the compounds, the compound combination, or the salt of the present disclosure on immune cell infiltration or proliferation, their effects in inhibiting the proliferation of CD4-positive and CD8-positive T cells were further analyzed.

First, a certain amount of human peripheral blood mononuclear cells (PBMCs) were incubated with 5 µM CellTrace cell proliferation assay reagent in an incubator at 37 °C with 5% CO₂ in the dark for 20 min. Then, 1.5 × 10⁵ CellTrace-stained PBMCs were seeded in a 96-well U-bottom plate and treated with Anti-CD3/CD28 antibody-coupled magnetic beads at a ratio of 1:1 (cell count:magnetic bead count) to induce differentiation into T cells. Subsequently, various compounds at corresponding concentrations (compound Y: 100 µM; compound T: 100 µM; compound combination Y + T: 100 µM compound Y + 100 µM compound T; YT salt: 100 µM; control group: UDCA (100 µM)) were co-incubated with the above-treated cells in the incubator at 37 °C with 5% CO₂ for 72 h, followed by analysis by a flow cytometer. Specifically, the test cells were first treated with a Live/dead dye (633 nm, 1:1000 dilution) at 4 °C for 30 min to distinguish dead cells from live cells. Subsequently, the above test cells were simultaneously co-incubated with wavelength dyes anti-CD45-PerCPCy5.5, anti-CD4-AF700, and anti-CD8-FITC at room temperature for 30 min to distinguish different series of T cells (CD4-positive T cells and CD8-positive T cells). After appropriate washing, the above-stained cells were analyzed by a flow cytometer at different wavelengths. Subsequent cell analysis refers to the analysis of live cells that tested positive after staining with the Live/dead dye. CD4-positive T cells refer to cells positive for staining with anti-CD45-PerCPCy5.5 and anti-CD4-AF700 dyes; CD8-positive T cells refer to cells positive for staining with anti-CD45-PerCPCy5.5 and anti-CD8-FITC dyes. Proliferation of CD4-positive and CD8-positive T cells was determined by the fluorescence of CellTrace dye.

As shown in FIG. 2, compared to the control group (DMSO, waveform diagram 1; from top to bottom: waveform diagrams 1-6), the peaks of CD4-positive T cell proliferation in the groups treated with the YT salt (waveform diagram 2) and the corresponding doses of compound Y (waveform diagram 3) and the compound combination Y + T (waveform diagram 5) exhibited an overall rightward shift, indicating that the proliferation of CD4-positive T cells in these groups was significantly inhibited. Similarly, as shown in FIG. 3, compared to the control group (DMSO, waveform diagram 1), the peaks of CD8-positive T cell proliferation in the groups treated with the YT salt (waveform diagram 2) and the corresponding doses of compound Y (waveform diagram 3) and the compound combination Y + T (waveform diagram 5) exhibited an overall rightward shift, indicating that the proliferation of CD8-positive T cells in these groups was significantly inhibited.

The above results show that, for the CD4-positive and CD8-positive T cells derived from Anti-CD3/CD28-induced differentiation of human PBMCs, the YT salt and the compound combination Y + T had significant inhibitory effects on the proliferation of CD4-positive and CD8-positive T cells, and their effects were significantly superior to those of compound T and compound U (UDCA), indicating that the YT salt and the compound combination Y + T possess potential inhibitory effects on immune cell infiltration or proliferation in neurodegenerative diseases.

### Example 5. Effect of Compound YT Salt on Onset of Disease, Severe Conditions, and Survival of SOD1^{G93A} ALS Mouse Model

The therapeutic effect of the YT salt on the survival status of ALS animals was evaluated in SOD1^{G93A} transgenic mice (B6SJL-Tg (SOD1*G93A) 1/cyagen). Sixteen female and fourteen male B6SJL-Tg(SOD1*G93A) 1/cyagen mice were selected and randomly divided into 2 groups (7 males + 8 females/group) according to sex and body weight, namely a model group (control) and a YT salt group (treatment group). The YT salt was intragastrically administered at a dose of 496 mg/kg once daily. The administration began on day 66 of mouse age. Body weight changes were monitored, animal status and symptom severity were evaluated, and individual survival periods were recorded. Statistics on the severity of animal symptoms included the time to the first onset of disease and the time to the first observation of severe conditions in the animals. In this study, "first onset of disease" was defined as the starting point of ALS onset when mice exhibited circling, abnormal gait, weakness in forelimbs or hindlimbs, tremors, or other manifestations. In this study, "severe condition" was defined as a state in which the animal was completely paralyzed and unable to move freely, but still alive; if the animal died directly without going through a "paralysis" stage after the onset of disease, the death was considered the occurrence of a severe condition. The survival period was statistically defined as the survival time of the animal at the endpoint of the test (day 130). The hazard ratio was calculated using the Log-rank test method.

The observation statistics of animal status are shown in FIG. 4. As can be seen from the analysis of the onset of disease, there was no statistical difference (P = 0.0802) in the time of first onset between the YT salt group and the model group. However, from the perspective of the overall onset of disease, the onset time in the YT salt group occurred after 90 days of mouse age, never earlier than that in the model group, and its median onset time (109 days) was 7 days longer than that in the model group (102 days); the hazard ratio for disease onset in the YT salt group (0.5716) was about 3 times lower than that in the model group (1.749). The test results show that the YT salt had the effects of delaying the onset time and reducing the hazard ratio for disease onset in the ALS model mice.

As can be seen from the results of the occurrence of severe conditions in the animals, as shown in FIG. 5, the incidence of severe conditions in the YT salt group was significantly lower than that in the model group (P < 0.05); the endpoint severe condition rate in the YT salt group was 66.667%; the median time to severe condition occurrence in the YT salt group was also significantly later than that in the model group; the median time to severe condition occurrence in the YT salt group (117 days) was about 8 days longer than that in the model control group (109 days); the hazard ratio for severe conditions in the YT salt group (0.45) was about 5 times lower than that in the model group (2.222). The test results show that the YT salt had the effect of effectively reducing the occurrence of severe conditions in the ALS model mice.

In the survival period statistics, as shown in FIG. 6, the survival period of animals in the YT salt group was significantly superior to that in the model group (P < 0.01); the endpoint survival rate of the YT salt group was 53.333%, while that of the model group was only 7.143%; the median survival period of the model group was 115.5 days, whereas in the YT salt group, the number of dead individuals had not exceeded half by the endpoint of the test, thus failing to meet the condition for calculating the median survival period; the death hazard ratio of the YT salt group (0.3212) was nearly 10 times lower than that of the model group (3.113). The test results show that the YT salt had the effects of significantly extending the survival period and reducing the death risk in the ALS model mice.

In conclusion, in the SOD1 G93A mutant ALS model mice, compared to the model group, the YT salt showed a trend of delaying the onset of disease, and could significantly reduce the incidence of severe conditions and extend their survival period. This suggests that the YT salt has a potential effect of preventing, alleviating, or treating ALS.

### Example 6. Effect of Compound YT Salt on MPTP-Induced Parkinson's Disease Mouse Model

Sixty male C57BL/6 mice were selected and randomly divided into 5 groups (12 mice/group), namely a sham surgery group (normal control), a model group (model control), an L-DOPA group (positive drug control, 40 mg/kg), a low-dose YT salt treatment group (167.5 mg/kg), and a high-dose YT salt treatment group (248 mg/kg). The mice were intraperitoneally injected with MPTP at a dose of 30 mg/kg for 5 consecutive days for modeling. The YT salt was intragastrically administered twice daily (with an interval of 7-8 h between two administrations) for 13 consecutive days. L-DOPA was administered twice daily (with an interval of 7-8 h between two administrations) for 8 consecutive days. The amelioration effect of the YT salt on the motor function of the MPTP-induced Parkinson's disease mice was evaluated by behavioral indicators such as a grip strength test and a rotarod test. Histopathological examination was performed on the animals to evaluate the protective effect of the YT salt on dopaminergic neurons in the MPTP-induced Parkinson's disease mice.

The grip strength test results, as shown in FIG. 7, indicate that the grip strength of the animals was significantly decreased after MPTP modeling (model group vs. sham surgery group, *P* < 0.0001). Under the designated treatment regimen, the L-DOPA group and the high-dose YT salt treatment group could significantly ameliorate the MPTP-induced decrease in animal grip strength (L-DOPA group vs. model group, *P <* 0.001; high-dose YT salt treatment group vs. model group, *P <* 0.01).

The rotarod test results, as shown in FIG. 8, indicate that after MPTP modeling, the median time on the rod in the rotarod test for animals in the model group was shorter than that in the sham surgery group. Under the designated treatment regimen, the high-dose YT salt treatment group had a significant amelioration effect on the MPTP-induced reduction in the animals' time on the rod (high-dose YT salt treatment group vs. model group, P < 0.05).

The results of TH immunohistochemical staining in the substantia nigra region, as shown in FIG. 9, indicate that compared to the sham surgery group, the number of TH-positive cells in the substantia nigra region of the model group was significantly reduced (*P* < 0.05), and the L-DOPA group, the low-dose YT salt treatment group, and the high-dose YT salt treatment group showed significant inhibition of the reduction in TH-positive cells in the substantia nigra region of the model animals at the designated doses (*P* < 0.0001).

The results of TH immunohistochemical staining in the striatum, as shown in FIG. 10, indicate that compared to the sham surgery group, the loss of TH-positive neurons in the model group was extremely significant (*P* < 0.0001), and the high-dose YT salt treatment group showed significant inhibition of the loss of TH-positive neurons in the substantia nigra region under the designated treatment regimen (*P* < 0.01).

The evaluation results of this example show that the YT salt at a dose of 167.5 mg/kg could significantly inhibit the apoptosis of dopaminergic neurons in the substantia nigra region and striatal region of the model animals; the YT salt at a dose of 248 mg/kg could not only significantly inhibit the apoptosis of dopaminergic neurons in the substantia nigra region of the model animals, but also significantly improve behavioral indicators such as grip strength and rotarod performance of the model animals. This suggests that the YT salt has a potential effect of preventing, alleviating, or treating Parkinson's disease.

### Example 7. Effect of Compound YT Salt on 6-OHDA-Induced Parkinson's Disease Rat Model

Forty-eight male SD rats were selected and divided into 4 groups (12 rats/group), namely a sham surgery group (normal control), a model group (model control), an istradefylline group (istradefylline, as a positive drug control, 10 mg/kg), and a YT salt treatment group (248 mg/kg). The Parkinson's disease model was established by injecting 20 µg of 6-OHDA into the right intracranial medial forebrain bundle (MFB) of rats. After modeling, istradefylline and the YT salt were administered intragastrically once daily for 6 consecutive weeks, and a grid walking test was performed at the end of the 6-week administration period to evaluate the disease progression in the rats.

The results, as shown in FIGs. 11 and 12, indicate that after the Parkinson's disease model was established by 6-OHDA, both the number of foot faults and the foot fault rate in the grid walking test were significantly increased in the animals of the model group (P < 0.05). Compared to the model group, the YT salt treatment group showed a significant reduction in the number of foot faults and the foot fault rate at the designated dose (*P* < 0.05).

The evaluation results of this example show that the YT salt had a significant amelioration effect on the motor behavior impairment of the 6-OHDA-induced Parkinson's disease rat model.This suggests that the YT salt has a potential effect of alleviating or treating Parkinson's disease.

It should be understood that various modifications or changes may be made by those skilled in the art after reading the aforementioned content of the present disclosure, and these equivalent forms also fall within the scope defined by the appended claims of the present application.

## Claims

1. A combination product, **characterized in that** the combination product comprises:
(a) a compound of formula (I) or a pharmaceutically acceptable salt, a solvate, a hydrate, or a stereoisomer thereof: 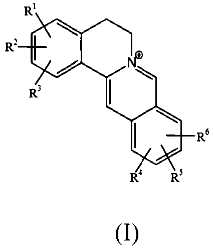
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N; and
(b) a bile acid or a derivative or an analog thereof, or a pharmaceutically acceptable salt, a solvate, a hydrate, or a stereoisomer thereof;
wherein the molar ratio of the component (a) to the component (b) is 1:1000-1000:1.

2. The combination product according to claim 1, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

3. The combination product according to claim 1 or 2, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

4. The combination product according to any one of claims 1-3, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

5. The combination product according to any one of claims 1-4, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

6. The combination product according to any one of claims 1-5, **characterized in that** the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate, preferably a maleate or a hydrochloride, and more preferably a hydrochloride.

7. The combination product according to any one of claims 1-6, **characterized in that** the compound of formula (I) is selected from:

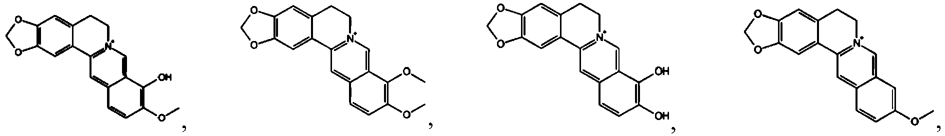

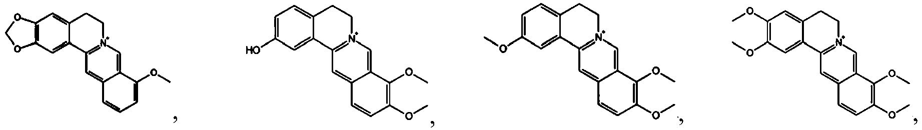

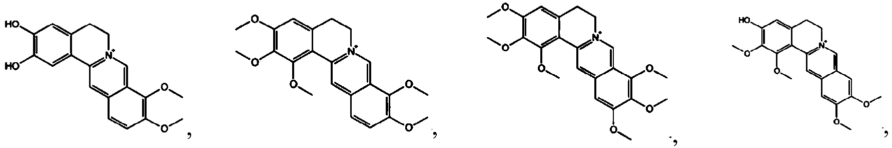

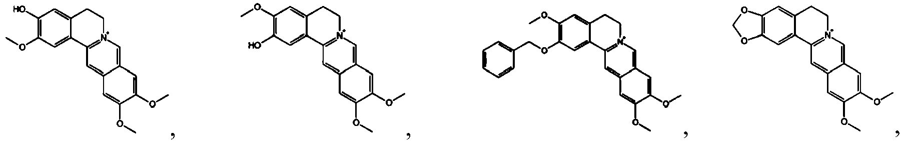

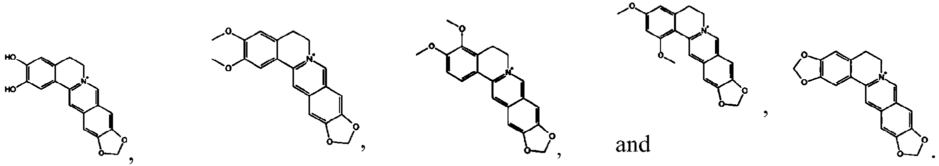

8. The combination product according to any one of claims 1-7, **characterized in that** the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

9. The combination product according to any one of claims 1-8, wherein the molar ratio of the component (a) to the component (b) is 1:1 or 2:1.

10. The combination product according to any one of claims 1-9, **characterized in that** the combination product is for use in improving the viability of cells, particularly neuronal cells, especially by reducing active oxidative metabolite-mediated oxidative damage in the cells, modulating redox homeostasis in the cells, or reducing mitochondrial dysfunction in the cells.

11. The combination product according to any one of claims 1-10, **characterized in that** the combination product is for use in treating, alleviating, and/or preventing a neurodegenerative disease or disorder.

12. The combination product according to claims 1-11, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

13. A pharmaceutical composition, **characterized in that** the pharmaceutical composition comprises:
(a) a compound of formula (I) or a pharmaceutically acceptable salt, a solvate, a hydrate, or a stereoisomer thereof: 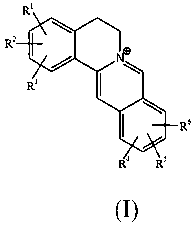
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N;
(b) a bile acid or a derivative or an analog thereof, or a pharmaceutically acceptable salt, a solvate, a hydrate, or a stereoisomer thereof; and
(c) a pharmaceutically acceptable carrier, excipient, and/or diluent;
wherein the molar ratio of the component (a) to the component (b) is 1: 1000-1000: 1.

14. The pharmaceutical composition according to claim 13, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

15. The pharmaceutical composition according to claim 13 or 14, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

16. The pharmaceutical composition according to any one of claims 13-15, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

17. The pharmaceutical composition according to any one of claims 13-16, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

18. The pharmaceutical composition according to any one of claims 13-17, **characterized in that** the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate, preferably a maleate or a hydrochloride, and more preferably a hydrochloride.

19. The pharmaceutical composition according to any one of claims 13-18, **characterized in that** the compound of formula (I) is selected from:

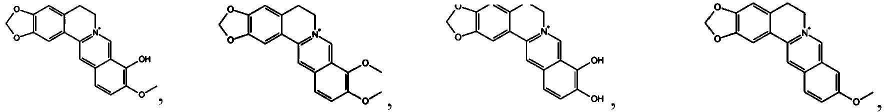

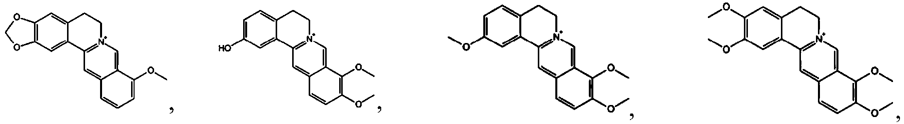

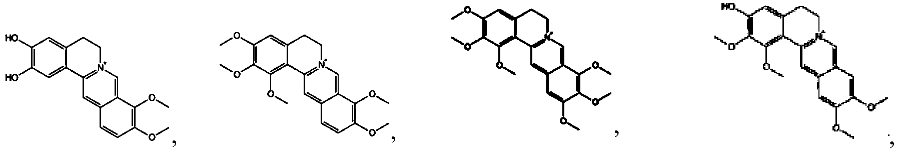

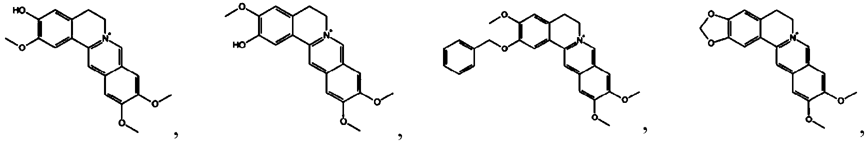

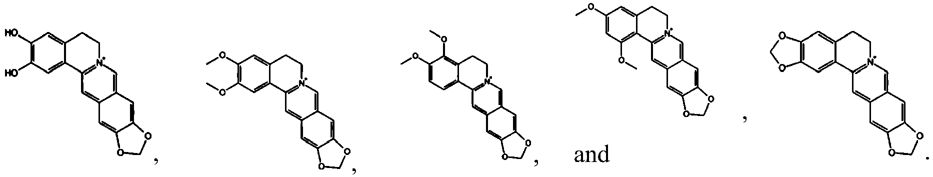

20. The pharmaceutical composition according to any one of claims 13-19, **characterized in that** the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

21. The pharmaceutical composition according to any one of claims 13-20, **characterized in that** the molar ratio of the component (a) to the component (b) is 1:1 or 2:1.

22. The pharmaceutical composition according to any one of claims 13-21, **characterized in that** the pharmaceutical composition is for use in improving the viability of cells, particularly neuronal cells, especially by reducing active oxidative metabolite-mediated oxidative damage in the cells, modulating redox homeostasis in the cells, or reducing mitochondrial dysfunction in the cells.

23. The pharmaceutical composition according to any one of claims 13-22, **characterized in that** the pharmaceutical composition is for use in treating, alleviating, and/or preventing a neurodegenerative disease or disorder.

24. The pharmaceutical composition according to claim 23, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

25. An acid-base addition salt of formula (II):
(A⁺)ₘ(B⁻)ₙ(C⁻)ₚ (II)
**characterized in that** (a) A⁺ is a cationic moiety, which is a compound of formula (I): 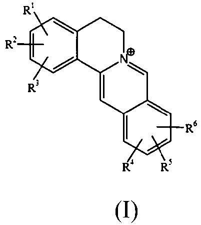
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N;
(b) B⁻ is an anionic moiety, which is a bile acid or a derivative or an analog thereof; and
(c) C⁻ is an acid anion;
wherein m, n, and p are each independently an integer selected from 1-6, such that the configuration of the salt reaches charge balance, and when m = n, p is 0.

26. The acid-base addition salt according to claim 25, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

27. The acid-base addition salt according to claim 25 or 26, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

28. The acid-base addition salt according to any one of claims 25-27, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

29. The acid-base addition salt according to any one of claims 25-28, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

30. The acid-base addition salt according to any one of claims 25-29, **characterized in that** the compound of formula (I) is a cationic moiety selected from the group consisting of:

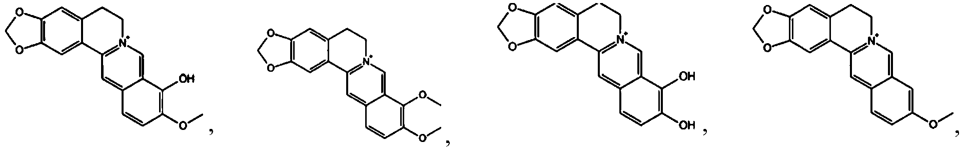

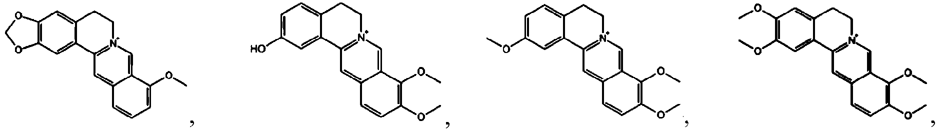

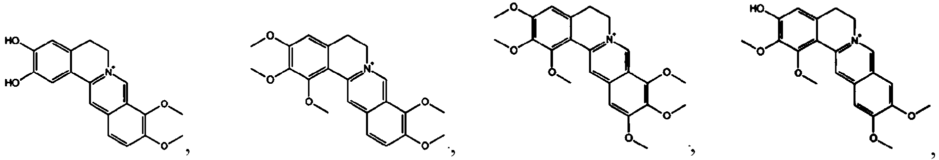

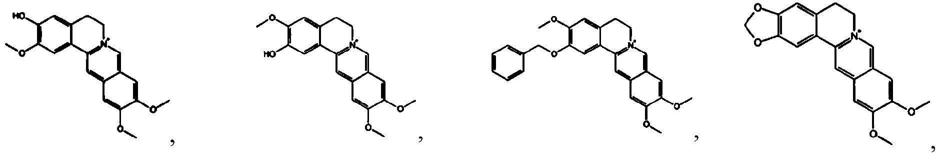

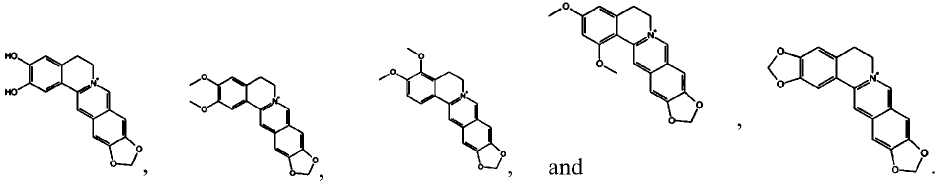

31. The acid-base addition salt according to any one of claims 25-30, **characterized in that** the component (b) is an anionic moiety selected from the group consisting of: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

32. The acid-base addition salt according to any one of claims 25-31, **characterized in that** m is 1, n is 1, and p is 0; or m is 2, n is 1, and p is 1.

33. The acid-base addition salt according to any one of claims 25 to 32, **characterized in that** the acid-base addition salt is for use in improving the viability of cells, particularly neuronal cells, especially by reducing active oxidative metabolite-mediated oxidative damage in the cells, modulating redox homeostasis in the cells, or reducing mitochondrial dysfunction in the cells.

34. The acid-base addition salt according to any one of embodiments 25-33, **characterized in that** the acid-base addition salt is for use in treating, alleviating, and/or preventing a neurodegenerative disease or disorder.

35. The acid-base addition salt according to claim 34, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

36. A pharmaceutical composition, **characterized in that** the pharmaceutical composition comprises the combination product according to any one of claims 1-12 or the acid-base addition salt according to any one of claims 25-35.

37. A method for treating, alleviating, and/or preventing a neurodegenerative disease or disorder, **characterized in that** the method comprises administering to a patient in need thereof the combination product, the pharmaceutical composition, the acid-base addition salt, or the compound of formula (I) or the pharmaceutically acceptable salt, the solvate, the hydrate, or the stereoisomer thereof according to any one of claims 1-36.

38. The method according to claim 37, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

39. A method for treating and/or preventing a neurodegenerative disease or disorder, **characterized in that** the method comprises:
regimen 1: administering to a patient in need thereof:
(a) a therapeutically and/or prophylactically effective amount of a compound of formula (I) or a pharmaceutically acceptable salt, a solvate, a hydrate, or a stereoisomer thereof: 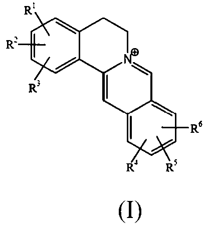
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N; or
regimen 2: administering to a patient in need thereof the following components simultaneously, concurrently, separately, or sequentially:
(a) a therapeutically and/or prophylactically effective amount of a compound of formula (I) or a pharmaceutically acceptable salt, a solvate, a hydrate, or a stereoisomer thereof: 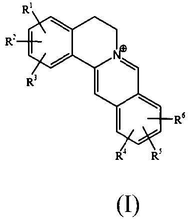
wherein R¹, R², R³, R⁴, R⁵, and R⁶ are identical or different, and are each independently selected from: hydrogen, halogen, hydroxyl, C₁-C₆ alkyl, C₁-C₆ haloalkyl, C₁-C₆ alkoxy, and C₁-C₆ haloalkoxy, wherein the hydroxyl, C₁-C₆ alkyl, C₁-C₆ haloalkyl, C₁-C₆ alkoxy, and C₁-C₆ haloalkoxy are optionally substituted with one or more groups selected from the group consisting of: halogen, C₁-C₆ alkyl, and phenyl;
or any two adjacent ones of R¹, R², and R³, together with the carbon atoms to which they are attached, form a 4- to 7-membered heterocyclic ring containing one or more heteroatoms selected from O, S, and N;
or any two adjacent ones of R⁴, R⁵, and R⁶, together with the carbon atoms to which they are attached, form a 4- to 7-membered heterocyclic ring containing one or more heteroatoms selected from O, S, and N; and
(b) a therapeutically and/or prophylactically effective amount of a bile acid or a derivative or an analog thereof, or a pharmaceutically acceptable salt, a solvate, a hydrate, or a stereoisomer thereof.

40. The method according to claim 39, **characterized in that** R¹, R², and R³ are each independently selected from: hydrogen, hydroxyl, methoxy, and benzyloxy; or any two adjacent ones of R¹, R², and R³, together with the carbon atoms to which they are attached, form a 4- to 7-membered heterocyclic ring containing one or more oxygen atoms.

41. The method according to claim 39 or 40, **characterized in that** R⁴, R⁵, and R⁶ are each independently selected from: hydrogen, hydroxyl, and methoxy; or any two adjacent ones of R⁴, R⁵, and R⁶, together with the carbon atoms to which they are attached, form a 4- to 7-membered heterocyclic ring containing one or more oxygen atoms.

42. The method according to any one of claims 39-41, **characterized in that** one or two of R¹, R², and R³ are hydrogen; or any two adjacent ones of R¹, R², and R³, together with the carbon atoms to which they are attached, form a 5-membered heterocyclic ring containing one or more oxygen atoms.

43. The method according to any one of claims 39-42, **characterized in that** one or two of R⁴, R⁵, and R⁶ are hydrogen; or any two adjacent ones of R⁴, R⁵, and R⁶, together with the carbon atoms to which they are attached, form a 5-membered heterocyclic ring containing one or more oxygen atoms.

44. The method according to any one of claims 39-43, **characterized in that** the pharmaceutically acceptable salt of the compound of formula (I) is selected from a maleate, a hydrochloride, an oxalate, a tartrate, a fumarate, a citrate, a malate, an adipate, a methanesulfonate, a phosphate, an acetate, a mandelate, and a sulfate, preferably a maleate or a hydrochloride, and more preferably a hydrochloride.

45. The method according to any one of claims 39-44, **characterized in that** the compound of formula (I) is selected from: 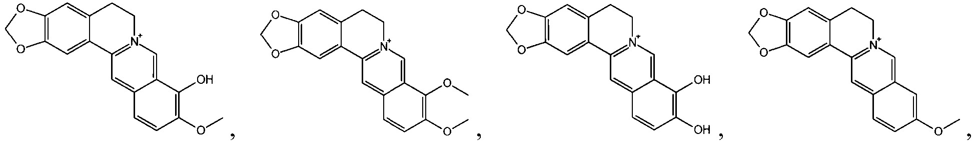 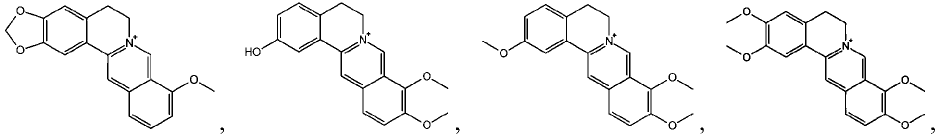 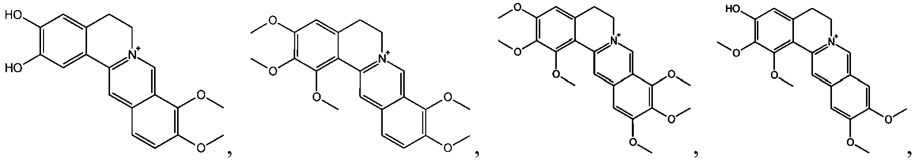 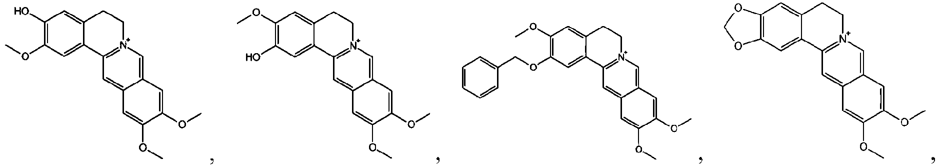 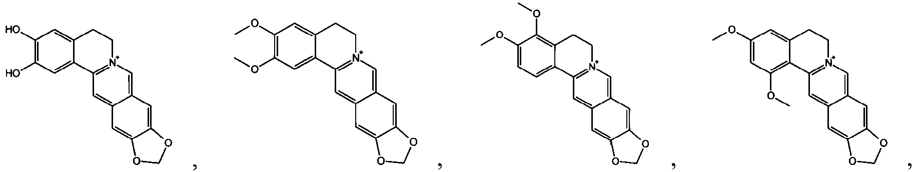 and

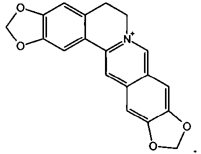

46. The method according to any one of claims 39-45, **characterized in that** the bile acid or the derivative or the analog thereof is selected from: cholic acid, obeticholic acid, ursodeoxycholic acid, chenodeoxycholic acid, hyodeoxycholic acid, 7-oxolithocholic acid, lithocholic acid, iododeoxycholic acid, iocholic acid, taurochenodeoxycholic acid, taurodeoxycholic acid, glycoursodeoxycholic acid, taurocholic acid, glycocholic acid, 24-norursodeoxycholic acid, and tauroursodeoxycholic acid.

47. The method according to any one of claims 39-46, **characterized in that** the molar ratio of the component (a) to the component (b) is 1:1 or 2:1.

48. The method according to any one of claims 39-47, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

49. A kit, **characterized in that** the kit comprises:
the combination product, the pharmaceutical composition, the acid-base addition salt, or the compound of formula (I) or the pharmaceutically acceptable salt, the solvate, the hydrate, or the stereoisomer thereof according to any one of claims 1-36, and instructions for use,
wherein the kit is used for treating, alleviating, or preventing a neurodegenerative disease or disorder.

50. The kit according to embodiment 49, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.

51. A method for improving the viability of cells, particularly neuronal cells, especially by reducing active oxidative metabolite-mediated oxidative damage in the cells, modulating redox homeostasis in the cells, or reducing mitochondrial dysfunction in the cells, **characterized in that** the method comprises contacting the cells with the combination product, the pharmaceutical composition, the acid-base addition salt, or the compound of formula (I) or the pharmaceutically acceptable salt, the solvate, the hydrate, or the stereoisomer thereof according to any one of claims 1-36.

52. A compound of formula (I) or a pharmaceutically acceptable salt, a solvate, a hydrate, or a stereoisomer thereof, **characterized in that** the compound of formula (I) or the pharmaceutically acceptable salt, the solvate, the hydrate, or the stereoisomer thereof is for use in improving the viability of cells, particularly neuronal cells, especially by reducing active oxidative metabolite-mediated oxidative damage in the cells, modulating redox homeostasis in the cells, or reducing mitochondrial dysfunction in the cells.

53. A compound of formula (I) or a pharmaceutically acceptable salt, a solvate, a hydrate, or a stereoisomer thereof, **characterized in that** the compound of formula (I) or the pharmaceutically acceptable salt, the solvate, the hydrate, or the stereoisomer thereof is for use in treating, alleviating, and/or preventing a neurodegenerative disease or disorder.

54. The compound of formula (I) or the pharmaceutically acceptable salt, the solvate, the hydrate, or the stereoisomer thereof according to claim 53, **characterized in that** the neurodegenerative disease or disorder is selected from Alzheimer's disease (AD), Huntington's disease (HD), Parkinson's disease (PD), amyotrophic lateral sclerosis (ALS), sarcopenia, muscular dystrophy, Pick's disease, multi-infarct dementia, Creutzfeldt-Jakob disease, dementia with Lewy bodies (DLB), mixed dementia, and frontotemporal dementia.
